# EUROPEAN PATENT SPECIFICATION

(11) **EP 2 814 466 B1**
(45) Date of publication and mention of the grant of the patent: **26.12.2018**
(21) Application number: 13708539.5
(22) Date of filing: 14.02.2013
(51) Int. Cl.: A61K 9/20, A61K 9/28, A61K 31/5377

(54) **TABLET COMPRISING 1-(4-METHOXYBUTYL)-N-(2-METHYLPROPYL)-N-[(3S,5R)-5-(MORPHOLIN-4-YLCARBONYL)PIPERIDIN-3-YL]-1H-BENZIMIDAZOLE-2-CARBOXAMIDE OR A SALT THEREOF**
TABLETTE ENTHALTEND 1-(4-METHOXYBUTYL)-N-(2-METHYLPROPYL)-N-[(3S,5R)-5-(MORPHOLIN-4-YLCARBONYL)PIPERIDIN-3-YL]-1H-BENZIMIDAZOLE-2-CARBOXAMID ODER EIN SALZ DAVON
COMPRIMÉ COMPRENANT 1-(4-METHOXYBUTYL)-N-(2-METHYLPROPYL)-N-[(3S,5R)-5-(MORPHOLIN-4-YLCARBONYL)PIPERIDIN-3-YL]-1H-BENZIMIDAZOLE-2-CARBOXAMIDE OU SON SEL

(30) Priority: 15.02.2012 JP 2012030987
(43) Date of publication of application: 24.12.2014
(73) Proprietor: Takeda Pharmaceutical Company Limited, Osaka-shi, Osaka 541-0045 (JP)
(72) Inventor: DEHARU, Yuko, Osaka-shi Osaka 532-0024 (JP)
(74) Representative: König, Gregor Sebastian
(86) International application number: PCT/JP2013/054246
(87) International publication number: WO 2013/122260

(56) References cited:
- EP-A1- 1 897 558
- WO-A2-2009/154300
- CA-A1- 2 802 483

## Description

### TECHNICAL FIELD OF THE INVENTION

The present invention relates to a tablet comprising the following (i)-(iii):
(i) 1-(4-methoxybutyl)-N-(2-methylpropyl)-N-[(3S,5R)-5-(morpholin-4-ylcarbonyl)piperidin-3-yl]-1H-benzimidazole-2-carboxamide or a salt thereof,
(ii) 30 to 50 wt% of crystalline cellulose in the tablet (of the weight of one tablet), and
(iii) 5 to 20 wt% of low-substituted hydroxypropylcellulose in the tablet (of the weight of one tablet).

### (Background of the Invention)

Patent document 1 (WO2009/154300) describes 1-(4-methoxybutyl)-N-(2-methylpropyl)-N-[(3S,5R)-5-(morpholin-4-ylcarbonyl)piperidin-3-yl]-1H-benzimidazole-2-carboxamide and a salt thereof, which have a renin inhibitory action and are useful as prophylactic or therapeutic agents for hypertension or various organ disorders caused by hypertension and the like.

Patent document 2 (WO2011/158880) improves hygroscopicity of 1-(4-methoxybutyl)-N-(2-methylpropyl)-N-[(3S,5R)-5-(morpholin-4-ylcarbonyl)piperidin-3-yl]-1H-benzimidazole-2-carboxamide hydrochloride by crystallizing the compound.

Patent document 3 (WO2010/013823) improves stability of a nonpeptidic compound having a primary or secondary amino group, which is contained as a pharmaceutically active ingredient in a tablet, by adding an acidic compound such as organic acid and the like.

Patent document 4 (JP-A-2008-94845) improves the shelf-life stability and resistance to degradation of a tablet containing a purine compound as a pharmaceutically active ingredient by adding, as excipients, a ductile filler and/or a brittle filler and a disintegrant, and forming a light-protective layer on the tablet.

Patent document 5 (JP-A-H11-243907) improves hardness and hygroscopicity of a tablet containing xylitol, by directly compressing a xylitol powder coated with a lipid, which lipid is solid at room temperature, and producing the tablet.

Patent document 6 (CA 2,802,483 A1) discloses a crystal of 1-(4-methoxybutyl)-N-(2-methylpropyl)-N-[(3S,5R)-5-(morpholin-4-ylcarbonyl)piperidin-3-yl]-1H-benzimidazole-2-carboxamide hydrochloride and tablets comprising the crystal. The crystal is disclosed to have decreased hygroscopicity and superior stability.

### [Document List]

### [patent documents]

patent document 1: WO 2009/154300
patent document 2: WO 2011/158880
patent document 3: WO 2010/013823
patent document 4: JP-A-2008-94845
patent document 5: JP-A-H11-243907
patent document 6: CA 2,802,483 A1

### SUMMARY OF THE INVENTION

### Problems to be Solved by the Invention

When a "compound showing deliquescence at a relative humidity of not less than 80% at 25°C" is used as a pharmaceutically active ingredient, a tablet which is easily produced and handled, and can be preserved for a long term can be provided once the hygroscopicity of the compound is improved or reduced.

### Means of Solving the Problems

The present inventors have conducted intensive studies in an attempt to solve the above-mentioned problems and found that addition of "an additive showing an equilibrium moisture absorption rate at relative humidity 75% at 25°C of not less than 7 wt% relative to its dry weight" to a tablet containing "a compound showing deliquescence at a relative humidity of not less than 80% at 25°C" as a pharmaceutically active ingredient improves (reduces) hygroscopicity of the compound, which resulted in the completion of the present invention.

Accordingly, the present invention relates to a tablet as defined in the claims. The present specification further relates to:
[1] a tablet comprising a compound showing deliquescence at a relative humidity of not less than 80% at 25°C and an additive showing an equilibrium moisture absorption rate at relative humidity 75% at 25°C of not less than 7 wt% relative to its dry weight;
[2] the tablet of the above-mentioned [1], wherein the compound has a secondary amine;
[3] the tablet of the above-mentioned [1], wherein the compound is represented by the formula (I): 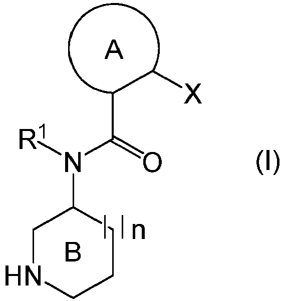 wherein
   R¹ is a hydrogen atom, an alkyl group optionally having substituent(s), an alkenyl group optionally having substituent(s), or a cycloalkyl group optionally having substituent(s);
   X is absent, or a hydrogen atom, an alkyl group optionally having substituent(s) or a cycloalkyl group optionally having substituent(s);
   ring A is a heterocycle optionally having substituent(s); ring B is a 5- to 7-membered, nitrogen-containing heterocycle optionally having substituent(s); and
   n is 0, 1 or 2 (hereinafter sometimes to be abbreviated as "Compound (I)" which includes compound (A) to be described later),
   or a salt thereof;
[4] the tablet of the above-mentioned [1], wherein the compound is 1-(4-methoxybutyl)-N-(2-methylpropyl)-N-[(3S,5R)-5-(morpholin-4-ylcarbonyl)piperidin-3-yl]-1H-benzimidazole-2-carboxamide (hereinafter sometimes to be abbreviated as "compound (A)") or a salt thereof;
[5] the tablet of the above-mentioned [4], wherein the 1-(4-methoxybutyl)-N-(2-methylpropyl)-N-[(3S,5R)-5-(morpholin-4-ylcarbonyl)piperidin-3-yl]-1H-benzimidazole-2-carboxamide or a salt thereof is 1-(4-methoxybutyl)-N-(2-methylpropyl)-N-[(3S,5R)-5-(morpholin-4-ylcarbonyl)piperidin-3-yl]-1H-benzimidazole-2-carboxamide hydrochloride;
[6] the tablet of the above-mentioned [1], wherein the additive comprises crystalline cellulose and low-substituted hydroxypropylcellulose;
[7] the tablet of the above-mentioned [6], wherein the content of the crystalline cellulose is 30 wt% or more of one tablet;
[8] the tablet of the above-mentioned [6], wherein the content of the low-substituted hydroxypropylcellulose is 5 wt% or more of one tablet;
[9] the preparation of the above-mentioned [1], wherein the content of the additive is 35 wt% or more of one tablet;
[10] the tablet of the above-mentioned [6], wherein the content of the crystalline cellulose is 30 wt% or more of one tablet, and the content of the low-substituted hydroxypropylcellulose is 5 wt% or more of one tablet;
[11] a tablet comprising the following (i)-(iii):
   (i) 1-(4-methoxybutyl)-N-(2-methylpropyl)-N-[(3S,5R)-5-(morpholin-4-ylcarbonyl)piperidin-3-yl]-1H-benzimidazole-2-carboxamide or a salt thereof,
   (ii) not less than 30 wt% of crystalline cellulose in the tablet (of the weight of one tablet), and
   (iii) not less than 5 wt% of low-substituted hydroxypropylcellulose in the tablet (of the weight of one tablet);
[12] the tablet of the above-mentioned [11], wherein 1-(4-methoxybutyl)-N-(2-methylpropyl)-N-[(3S,5R)-5-(morpholin-4-ylcarbonyl)piperidin-3-yl]-1H-benzimidazole-2-carboxamide or a salt thereof is 1-(4-methoxybutyl)-N-(2-methylpropyl)-N-[(3S,5R)-5-(morpholin-4-ylcarbonyl)piperidin-3-yl]-1H-benzimidazole-2-carboxamide hydrochloride;
and the like.

### Effect of the Invention

According to the present specification, a tablet containing a compound showing deliquescence at a relative humidity of not less than 80% at 25°C as a pharmaceutically active ingredient, which shows improved (reduced) hygroscopicity of the compound, can be easily produced and handled, and can be stably preserved for a long term, can be provided.

According to the present specification, a tablet containing a compound showing deliquescence at a relative humidity of not less than 80% at 25°C as a pharmaceutically active ingredient, which is stable and shows no change in the dissolution property even under high humidity, can be provided.

### BRIEF DESCRIPTION OF THE DRAWINGS

Fig. 1 shows an equilibrium moisture absorption rate of hydrochloride (monohydrochloride) of compound (A) at various relative humidities at 25°C, wherein the vertical axis "Weight (%)" shows the equilibrium moisture absorption rate (wt%) and the horizontal axis "RH (%)" shows relative humidity at 25°C.
Fig. 2 shows an equilibrium moisture absorption rate of hydrochloride (monohydrochloride) of compound (A) at various relative humidities at 25°C, wherein the vertical axis "Weight (%)" shows the equilibrium moisture absorption rate (wt%) and the horizontal axis "RH (%)" shows relative humidity at 25°C.
Fig. 3 shows an equilibrium moisture absorption rate of low-substituted hydroxypropylcellulose at various relative humidities at 25°C. The vertical axis "Weight (%)" shows an equilibrium moisture absorption rate (wt%) and the horizontal axis "RH (%)" shows relative humidity at 25°C.
Fig. 4 shows an equilibrium moisture absorption rate of xylitol at various relative humidities at 25°C. The vertical axis "Weight (%)" shows an equilibrium moisture absorption rate (wt%) and the horizontal axis "RH (%)" shows relative humidity at 25°C.

### (DETAILED DESCRIPTION OF THE INVENTION)

In the present specification, the "relative humidity" is a ratio in percentage of water vapor pressure at a given temperature to saturated water vapor pressure at said temperature.

In the present specification, the "equilibrium moisture absorption rate" is a ratio in percentage of an increase in the weight of a sample when it absorbed moisture at a given relative humidity at a given temperature and reached equilibrium, to the weight of the sample when dried completely (dry weight).

In the present specification, the "deliquescence" means the property of a solid to be a liquid as a result of dissolution into the absorbed water vapor in the air.

In the present specification, "high humidity" means relative humidity of not less than 75%.

In the present specification, the "dissolution property" means the property of a pharmaceutically active ingredient to dissolve from the preparation into a solution. The dissolution property can be evaluated by, for example, the Dissolution Test Method (e.g., rotating basket method, Paddle Method) described in the Japanese Pharmacopoeia 16th Ed.

Examples of the "halogen atom" in the present specification include fluorine, chlorine, bromine and iodine.

Examples of the "C₁₋₄ alkylenedioxy group" in the present specification include methylenedioxy, ethylenedioxy, trimethylenedioxy and the like.

Examples of the "alkyl group" in the present specification include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl and the like. Among these, a C₁₋₆ alkyl group is preferable.

Examples of the "alkenyl group" in the present specification include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl and the like. Among these, a C₂₋₆ alkenyl group is preferable.

Examples of the "alkynyl group" in the present specification include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like. Among these, a C₂₋₆ alkynyl group is preferable.

Examples of the "cycloalkyl group" in the present specification include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like. Among these, a C₃₋₁₀ cycloalkyl group is preferable.

Examples of the "cycloalkyl" moiety of the "cycloalkyloxy group" in the present specification include those similar to the above-mentioned "cycloalkyl group".

Examples of the "alkylthio group" in the present specification include methylthio, ethylthio, propylthio, isopropylthio, butylthio, isobutylthio, sec-butylthio, tert-butylthio, pentylthio, isopentylthio, neopentylthio, 1-ethylpropylthio, hexylthio, isohexylthio, 1,1-dimethylbutylthio, 2,2-dimethylbutylthio, 3,3-dimethylbutylthio, 2-ethylbutylthio and the like. Among these, a C₁₋₆ alkylthio group is preferable.

Examples of the "alkylsulfinyl group" in the present specification include methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, isopentylsulfinyl, neopentylsulfinyl, 1-ethylpropylsulfinyl, hexylsulfinyl, isohexylsulfinyl, 1,1-dimethylbutylsulfinyl, 2,2-dimethylbutylsulfinyl, 3,3-dimethylbutylsulfinyl, 2-ethylbutylsulfinyl and the like. Among these, a C₁₋₆ alkylsulfinyl group is preferable.

Examples of the "alkylsulfonyl group" in the present specification include methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, 1-ethylpropylsulfonyl, hexylsulfonyl, isohexylsulfonyl, 1,1-dimethylbutylsulfonyl, 2,2-dimethylbutylsulfonyl, 3,3-dimethylbutylsulfonyl, 2-ethylbutylsulfonyl and the like. Among these, a C₁₋₆ alkylsulfonyl group is preferable.

Examples of the "alkoxy group" in the present specification include methoxy, ethoxy, propoxy, isopropoxy, butoxy, isobutoxy, sec-butoxy, tert-butoxy, pentyloxy, isopentyloxy, neopentyloxy, 1-ethylpropyloxy, hexyloxy, isohexyloxy, 1,1-dimethylbutyloxy, 2,2-dimethylbutyloxy, 3,3-dimethylbutyloxy, 2-ethylbutyloxy and the like. Among these, a C₁₋₆ alkoxy group is preferable.

Examples of the "C₁₋₆ alkoxy-carbonyl group" in the present specification include methoxycarbonyl, ethoxycarbonyl, propoxycarbonyl, tert-butoxycarbonyl and the like.

Examples of the "C₁₋₆ alkyl-carbonyl group" in the present specification include acetyl, propanoyl, butanoyl, isobutanoyl, pentanoyl, isopentanoyl, hexanoyl and the like.

The "optionally halogenated" in the present specification means being optionally substituted by 1 to 5, preferably 1 to 3 halogen atoms.

Examples of the "C₁₋₆ alkylene group" in the present specification include methylene, ethylene, trimethylene, tetramethylene, pentamethylene, hexamethylene, -CH(CH₃)-,-C(CH₃)₂-, -CH(CH₂CH₃)-, -C(CH₂CH₃)₂-, -CH (CH₃)-CH₂-, -CH₂-CH(CH₃)-, -C(CH₃)₂-CH₂-, -CH₂-C(CH₃)₂-, -CH(CH₃)-(CH₂)₂-, -CH₂-CH(CH₃)-CH₂-, -(CH₂)₂-CH(CH₃)-, -C(CH₃)₂-(CH₂)₂-, -CH₂-C(CH₃)₂-CH₂-, -(CH₂)₂-C(CH₃)₂- and the like.

Examples of the "hydrocarbon group" of the "hydrocarbon group optionally having substituent(s)" in the present specification include an alkyl group, an alkenyl group, an alkynyl group, an alkylidene group, a cycloalkyl group, a cycloalkenyl group, a cycloalkadienyl group, an aryl group, an aralkyl group, an arylalkenyl group, a cycloalkylalkyl group and the like. Preferable examples include a C₁₋₁₀ alkyl group, a C₂₋₁₀ alkenyl group, a C₂₋₁₀ alkynyl group, a C₁₋₃ alkylidene group, a C₃₋₁₀ cycloalkyl group, a C₃₋₁₀ cycloalkenyl group, a C₄₋₁₀ cycloalkadienyl group, a C₆₋₁₄ aryl group, a C₇₋₁₆ aralkyl group, a C₈₋₁₃ arylalkenyl group, a C₃₋₁₀ cycloalkyl-C₁₋₆ alkyl group and the like. The above-mentioned C₃₋₁₀ cycloalkyl group, C₃₋₁₀ cycloalkenyl group and C₄₋₁₀ cycloalkadienyl group may be each condensed with a benzene ring.

Examples of the "C₁₋₁₀ alkyl group" in the present specification include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like. Among these, a C₁₋₆ alkyl group is preferable.

Examples of the "C₂₋₁₀ alkenyl group" in the present specification include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like. Among these, a C₂₋₆ alkenyl group is preferable.

Examples of the "C₂₋₁₀ alkynyl group" in the present specification include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like. Among these, a C₂₋₆ alkynyl group is preferable.

Examples of the "C₁₋₃ alkylidene group" in the present specification include methylidene, ethylidene, propylidene, isopropylidene and the like.

Examples of the "C₃₋₁₀ cycloalkyl group" in the present specification include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like. Among these, a C₃₋₆ cycloalkyl group is preferable. The above-mentioned C₃₋₁₀ cycloalkyl may be condensed with a benzene ring, and examples of the fused group include indanyl, tetrahydronaphthyl, fluorenyl and the like.

Examples of the "C₃₋₁₀ cycloalkenyl group" in the present specification include 2-cyclopenten-1-yl, 3-cyclopenten-1-yl, 2-cyclohexen-1-yl, 3-cyclohexen-1-yl and the like. The above-mentioned C₃₋₁₀ cycloalkenyl may be condensed with a benzene ring, and examples of the fused group include indenyl and the like.

Examples of the "C₄₋₁₀ cycloalkadienyl group" in the present specification include 2,4-cyclopentadien-1-yl, 2,4-cyclohexadien-1-yl, 2,5-cyclohexadien-1-yl and the like. The above-mentioned C₄₋₁₀ cycloalkadienyl may be condensed with a benzene ring.

Examples of the "C₆₋₁₄ aryl group" in the present specification include phenyl, 1-naphthyl, 2-naphthyl, biphenylyl, 2-anthryl and the like. Among these, a C₆₋₁₀ aryl group is preferable, and phenyl is more preferable. The above-mentioned C₆₋₁₄ aryl may be condensed with C₃₋₁₀ cycloalkane (examples of the C₃₋₁₀ cycloalkane include a ring corresponding to the above-mentioned C₃₋₁₀ cycloalkyl group), and examples of the fused group include tetrahydronaphthyl, indanyl and the like.

Examples of the "C₇₋₁₆ aralkyl group" in the present specification include benzyl, phenethyl, naphthylmethyl, biphenylylmethyl and the like.

Examples of the "C₈₋₁₃ arylalkenyl group" in the present specification include styryl and the like.

Examples of the "C₃₋₁₀ cycloalkyl-C₁₋₆ alkyl group" in the present specification include cyclopropylmethyl, cyclohexylmethyl and the like.

The "hydrocarbon group" of the "hydrocarbon group optionally having substituent(s)" optionally has substituent(s) (e.g., 1 to 5, preferably 1 to 3 substituents) at substitutable position(s). When the number of the substituents is two or more, respective substituents may be the same or different.

Examples of the "substituent" of the "hydrocarbon group optionally having substituent(s)" include the following substituents (hereinafter to be referred to as substituent group A).
(1) a halogen atom;
(2) a nitro group;
(3) a cyano (nitrile) group;
(4) a hydroxy group;
(5) an alkoxy group optionally having substituent(s) (particularly, C₁₋₆ alkoxy group optionally having substituent(s));
(6) an amino group optionally having substituent(s);
(7) =O (oxo group);
(8) =S (thioxo group);
(9) a mercapto group optionally having a substituent (the mercapto group is optionally oxidized);
(10) a C₁₋₄ alkylenedioxy group;
(11) an alkyl group optionally having substituent(s) (particularly, C₁₋₆ alkyl group optionally having substituent(s));
(12) a C₇₋₁₆ aralkyl group;
(13) an acyl group;
(14) a 3- to 10-membered cyclic hydrocarbon group optionally having substituent(s);
(15) a 3- to 10-membered heterocyclic group optionally having substituent(s) and the like.

Examples of the "3- to 10-membered cyclic hydrocarbon group" of the "3- to 10-membered cyclic hydrocarbon group optionally having substituent(s)" for substituent group A include C₃₋₁₀ cycloalkyl group, C₃₋₁₀ cycloalkenyl group, C₄₋₁₀ cycloalkadienyl group, C₆₋₁₀ aryl group and the like. Examples of the C₃₋₁₀ cycloalkyl group, C₃₋₁₀ cycloalkenyl group, C₄₋₁₀ cycloalkadienyl group and C₆₋₁₀ aryl group include those similar to the C₃₋₁₀ cycloalkyl group, C₃₋₁₀ cycloalkenyl group, C₄₋₁₀ cycloalkadienyl group and C₆₋₁₀ aryl group exemplified as the "hydrocarbon group" of the "hydrocarbon group optionally having substituent(s)".

Examples of the "3- to 10-membered heterocyclic group" of the "3- to 10-membered heterocyclic group optionally having substituent(s)" for substituent group A include a 3- to 10-membered ring from the "heterocyclic group" of the "heterocyclic group optionally having substituent(s)" to be mentioned later.

Examples of the "substituent" of the "alkyl group optionally having substituent(s)" and "alkoxy group optionally having substituent(s)", "3- to 10-membered cyclic hydrocarbon group optionally having substituent(s)" and "3- to 10-membered heterocyclic group optionally having substituent(s)" for substituent group A include 1 to 5, preferably 1 to 3 selected from the following substituents (hereinafter to be referred to as substituent group B). When the number of the substituents is two or more, the respective substituents may be the same or different.

(1) a halogen atom;
(2) a nitro group;
(3) a cyano (nitrile) group;
(4) a hydroxy group;
(5) a C₁₋₆ alkoxy group optionally having 1 to 3 halogen atoms;
(6) an amino group;
(7) a mono- or di-C₁₋₆ alkylamino group;
(8) a C₇₋₁₆ aralkylamino group;
(9) a C₁₋₆ alkoxy-carbonylamino group;
(10) a C₁₋₆ alkyl-carbonylamino group;
(11) a C₁₋₆ alkyl-carbonyloxy group;
(12) a C₁₋₆ alkyl-carbonyl group;
(13) a carboxy group;
(14) a C₁₋₆ alkoxy-carbonyl group;
(15) a carbamoyl group;
(16) a mono- or di-C₁₋₆ alkylcarbamoyl group;
(17) =O (oxo group);
(18) =S (thioxo group);
(19) a mercapto group;
(20) a C₁₋₆ alkylthio group;
(21) a C₁₋₆ alkylsulfinyl group;
(22) a C₁₋₆ alkylsulfonyl group;
(23) a C₁₋₄ alkylenedioxy group;
(24) a C₁₋₆ alkyl group optionally having 1 to 3 substituents selected from
   (a) a halogen atom,
   (b) a hydroxy group,
   (c) a C₁₋₆ alkoxy group,
   (d) a C₆₋₁₄ aryl group,
   (e) an amino group,
   (f) a mono- or di-C₁₋₆ alkylamino group,
   (g) a C₇₋₁₆ aralkylamino group, and
   (h) a C₁₋₆ alkoxy-carbonylamino group;
(25) an aryl group optionally having 1 to 3 halogen atoms;
(26) an aromatic heterocyclic group (e.g., pyridyl, pyrazolyl, triazolyl) optionally having 1 to 3 halogen atoms;
(27) a nonaromatic heterocyclic group (e.g., dioxolyl) optionally having 1 to 3 substituents selected from a C₁₋₆ alkyl group and an oxo group;
(28) a C₇₋₁₆ aralkyl group;
(29) a C₃₋₁₀ cycloalkyl group;
(30) a cyclic amino group (e.g., pyrrolidinyl, piperidino, morpholino, thiomorpholino, piperazinyl, imidazolidin-1-yl, pyrazolidin-1-yl etc.) optionally having an oxo group and the like.

Examples of the "substituent" of the "amino group optionally having substituent(s)" for substituent group A include 1 or 2 selected from substituent group B. When the number of the substituents is two, the respective substituents may be the same or different.

Examples of the "substituent" of the "mercapto group optionally having a substituent" for substituent group A include substituent group B. The mercapto group may be oxidized by 1 or 2 oxygens.

Examples of the "heterocyclic group" of the "heterocyclic group optionally having substituent(s)" in the present specification include an aromatic heterocyclic group and a nonaromatic heterocyclic group.

Examples of the "aromatic heterocyclic group" include a 4- to 10-membered (preferably 5- or 6-membered) monocyclic aromatic heterocyclic group containing, as a ring-constituting atom besides carbon atoms, 1 to 4 heteroatoms selected from an oxygen atom, a sulfur atom and a nitrogen atom, and a fused aromatic heterocyclic group. Examples of the fused aromatic heterocyclic group include a group derived from a fused ring wherein a ring corresponding to such 4- to 10-membered monocyclic aromatic heterocyclic group, and 1 or 2 rings selected from a 5- or 6-membered aromatic heterocycle containing 1 or 2 nitrogen atoms, a 5-membered aromatic heterocycle containing one sulfur atom and a benzene ring are condensed, and the like.

Examples of the "aromatic heterocyclic group" include 4- to 7-membered (preferably 5- or 6-membered) monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,2,4-thiadiazol-5-yl, 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,3,5-triazin-2-yl, 1,3,5-triazin-4-yl, 1,2,3-triazin-4-yl, 1,2,4-triazin-3-yl) and the like; fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridynyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridynyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like; and the like.

Examples of the "non-aromatic heterocyclic group" include a 3- to 10-membered (preferably 5- or 6-membered) monocyclic non-aromatic heterocyclic group containing, as a ring-constituting atom besides carbon atoms, 1 to 4 heteroatoms selected from an oxygen atom, a sulfur atom and a nitrogen atom, and a fused non-aromatic heterocyclic group. Examples of the fused non-aromatic heterocyclic group include a group derived from a fused ring wherein a ring corresponding to such 3- to 10-membered monocyclic non-aromatic heterocyclic group, and 1 or 2 rings selected from a 5- or 6-membered heterocycle containing 1 or 2 nitrogen atoms, a 5-membered heterocycle containing one sulfur atom and a benzene ring are condensed, and the like.

Examples of the "non-aromatic heterocyclic group" include 4- to 7-membered (preferably 5- or 6-membered) monocyclic non-aromatic heterocyclic groups such as pyrrolidinyl (e.g., 1-pyrrolidinyl, 2-pyrrolidinyl), piperidinyl (e.g., piperidino, 2-piperidinyl, 3-piperidinyl, 4-piperidinyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), hexahydropyrimidinyl (e.g., hexahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl) and the like; fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydrobenzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydrobenzofuran-3-yl), chromenyl (e.g., 4H-chromen-2-yl, 2H-chromen-3-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl) and the like; and the like.

The above-mentioned "heterocyclic group" optionally has substituent(s) (e.g., 1 to 5, preferably 1 to 3 substituents) at substitutable position(s). When the number of the substituents is two or more, the respective substituents may be the same or different.

Examples of the "substituent" of the "heterocyclic group optionally having substituent(s)" include the groups exemplified as the aforementioned substituent group A and the like.

Examples of the "acyl group" in the present specification include groups represented by the formulas: -COR^{A}, -CO-OR^{A},-SO₂R^{A}, -SOR^{A}, -CO-NR'R", -CS-NR'R" wherein R^{A} is a hydrogen atom, a hydroxy group, a hydrocarbon group optionally having substituent(s), an amino group optionally having substituent(s) or a heterocyclic group optionally having substituent(s). R' and R" are each a hydrogen atom, a hydrocarbon group optionally having substituent(s) or a heterocyclic group optionally having substituent(s), or R' and R" form, together with the nitrogen atom bonded thereto, a nitrogen-containing heterocycle optionally having substituent(s), and the like.

Examples of the "hydrocarbon group" of the "hydrocarbon group optionally having substituent(s)" for R^{A}, R' or R" include those similar to the "hydrocarbon group" of the aforementioned "hydrocarbon group optionally having substituent(s)".

Examples of the "heterocyclic group" of the "heterocyclic group optionally having substituent(s)" for R^{A}, R' or R" include those similar to the "heterocyclic group" of the aforementioned "heterocyclic group optionally having substituent(s)".

Examples of the "amino group optionally having substituent(s)" for R^{A} include those similar to the "amino group optionally having substituent(s)" of the aforementioned substituent group A.

Examples of the substituent of the "hydrocarbon group optionally having substituent(s)" and "heterocyclic group optionally having substituent(s)" for R^{A}, R' or R" each include 1 to 5, preferably 1 to 3, selected from the aforementioned substituent group A. When the number of the substituents is two or more, the respective substituents may be the same or different.

Examples of the "nitrogen-containing heterocycle" of the "nitrogen-containing heterocycle optionally having substituent(s)" formed by R' and R" together with the nitrogen atom bonded thereto include a 4- to 7-membered nonaromatic nitrogen-containing heterocycle containing, as a ring-constituting atom besides carbon atom, one nitrogen atom, and optionally further containing 1 or 2 hetero atoms selected from an oxygen atom, a sulfur atom and a nitrogen atom. The nonaromatic nitrogen-containing heterocycle may be condensed with a benzene ring.

Examples of the nitrogen-containing heterocycle include azetidine, pyrrolidine, imidazolidine, pyrazolidine, piperidine, homopiperidine, piperazine, homopiperazine, morpholine, homomorpholine, thiomorpholine, thiohomomorpholine, dihydrobenzoxazine (e.g., 3,4-dihydro-2H-1,4-benzoxazine), 1,2,3,4-tetrahydroquinoline, 7-aza-bicyclo[2.2.1]heptane and the like.

The "nitrogen-containing heterocycle" optionally has (preferably 1 to 3, more preferably 1 or 2) substituent(s) at substitutable position(s). Examples of the substituent include substituent group B and the like. When the number of the substituents is two or more, the respective substituents may be the same or different.

Preferable examples of the "acyl" include
(1) a formyl group;
(2) a carboxy group;
(3) a C₁₋₆ alkyl-carbonyl group;
(4) a C₁₋₆ alkoxy-carbonyl group (e.g., methoxycarbonyl, ethoxycarbonyl, propoxycarbonyl, tert-butoxycarbonyl) optionally having 1 to 3 substituents selected from the substituent group B;
(5) a group represented by the formula: -CO-NR'R"
   wherein R' and R" are each a hydrogen atom, a hydrocarbon group optionally having 1 to 3 substituents selected from the aforementioned substituent group B or a heterocyclic group optionally having 1 to 3 substituents selected from the aforementioned substituent group B, or R' and R" optionally form, together with the nitrogen atom bonded thereto, a nitrogen-containing heterocycle optionally having 1 to 3 substituents selected from the aforementioned substituent group B, and the like.

Examples of the "aryl group" in the present specification include C₆₋₁₄ aryl such as phenyl, 1-naphthyl, 2-naphthyl, biphenylyl, 2-anthryl and the like. Among these, C₆₋₁₀ aryl is preferable and phenyl is more preferable. The above-mentioned aryl may be condensed with C₃₋₁₀ cycloalkane (examples of the C_{3- 10} cycloalkane include a ring corresponding to the above-mentioned C₃₋₁₀ cycloalkyl), and examples of the fused group include tetrahydronaphthyl, indanyl and the like.

Examples of the "aryl" moiety of the "aryloxy group" in the present specification include those similar to the above-mentioned "aryl group".

Examples of the "heteroaryl group" in the present specification include a monocyclic aromatic heterocyclic group and a fused aromatic heterocyclic group from the "heterocyclic group" of the aforementioned "heterocycle optionally having substituent(s)".

Examples of the "heterocycle" in the present specification include monocyclic heterocycle and fused heterocycle.

Examples of the "monocyclic heterocycle" include monocyclic aromatic heterocycle and monocyclic non-aromatic heterocycle.

Examples of the "monocyclic aromatic heterocycle" include a 4- to 10-membered (preferably 5- or 6-membered) monocyclic aromatic heterocycle containing, as a ring-constituting atom besides carbon atoms, 1 to 4 heteroatoms selected from an oxygen atom, a sulfur atom and a nitrogen atom.

Examples of the "monocyclic aromatic heterocycle" include a 4- to 7-membered (preferably 5- or 6-membered) monocyclic aromatic heterocycle such as furan, thiophene, pyridine, pyrimidine, pyridazine, pyrazine, pyrrole, imidazole, pyrazole, thiazole, isothiazole, oxazole, isoxazole, oxadiazolyl (e.g., 1,2,4-oxadiazole, 1,3,4-oxadiazole), thiadiazole (e.g., 1,2,4-thiadiazole, 1,3,4-thiadiazole), triazole (e.g., 1,2,4-triazole, 1,2,3-triazole), tetrazole, triazine (e.g., 1,3,5-triazine, 1,2,3-triazine, 1,2,4-triazine) and the like.

Examples of the "monocyclic non-aromatic heterocycle" include a 3- to 10-membered (preferably 5- or 6-membered) monocyclic non-aromatic heterocycle containing, as a ring-constituting atom besides carbon atoms, 1 to 4 heteroatoms selected from an oxygen atom, a sulfur atom and a nitrogen atom.

Examples of the "monocyclic non-aromatic heterocycle" include a 4- to 7-membered (preferably 5- or 6-membered) monocyclic non-aromatic heterocycle such as pyrrolidine, piperidine, morpholine, thiomorpholine, piperazine, hexamethylenimine, oxazolidine, thiazolidine, imidazolidine, oxazoline, thiazoline, imidazoline, dioxole, dioxolane, dihydrooxadiazole (e.g., 4,5-dihydro-1,2,4-oxadiazole), 2-thioxo-1,3-oxazolidine, pyran, tetrahydropyran, thiopyran, tetrahydrothiopyran, 1-oxidetetrahydrothiopyran, 1,1-dioxidetetrahydrothiopyran, tetrahydrofuran, pyrazolidine, pyrazoline, tetrahydropyrimidine, dihydrotriazole, tetrahydrotriazole (e.g., 2,3,4,5-tetrahydro-1H-1,2,3-triazole) and the like.

Examples of the "fused heterocycle" include fused aromatic heterocycle and fused non-aromatic heterocycle.

Examples of the "fused aromatic heterocycle" include a ring wherein a 4- to 7-membered (preferably 5- or 6-membered) monocyclic aromatic heterocycle containing, as a ring-constituting atom besides carbon atoms, 1 to 4 heteroatoms selected from an oxygen atom, a sulfur atom and a nitrogen atom, and 1 or 2 rings selected from a 5- or 6-membered aromatic heterocycle containing 1 or 2 nitrogen atoms, a 5-membered aromatic heterocycle containing one sulfur atom and a benzene ring are condensed and the like.

Examples of the "fused aromatic heterocycle" include quinoline, isoquinoline, quinazoline, quinoxaline, benzofuran, benzothiophene, benzoxazole, benzisoxazole, benzothiazole, benzimidazole, benzotriazole, indole, indazole, pyrrolopyrazine (e.g., 1H-pyrrolo[2,3-b]pyrazine), imidazopyridine (e.g., 3H-imidazo[4,5-b]pyridine, 1H-imidazo[5,4-b]pyridine, 1H-imidazo[4,5-c]pyridine, imidazo[1,2-a]pyridine), imidazopyrazine (e.g., 1H-imidazo[4,5-b]pyrazine, imidazo[1,2-a]pyrazine), imidazopyrimidine (e.g., imidazo[1,2-a]pyrimidine, imidazo[1,2-c]pyrimidine), imidazopyridazine (e.g., imidazo[1,2-b]pyridazine), pyrazolopyridine (e.g., 1H-pyrazolo[4,3-c]pyridine), thienopyrrole (e.g., 4H-thieno[3,2-b]pyrrole), pyrazolothiophene (e.g., 2H-pyrazolo[3,4-b]thiophene), pyrazolotriazine (e.g., pyrazolo[5,1-c][1,2,4]triazine), pyrrolopyridine (e.g., 1H-pyrrolo[1,2-b]pyridine), 1,4-dihydropyrrolo[3,2-b]pyrrole, 4H-furo[3,2-b]pyrrole, 4H-thieno[3,2-b]pyrrole, 1H-furo[2,3-d]imidazole, 1H-thieno[2,3-d]imidazole and the like.

Examples of the "fused non-aromatic heterocycle" include a ring wherein a 4- to 7-membered (preferably 5- or 6-membered) monocyclic non-aromatic heterocycle containing, as a ring-constituting atom besides carbon atoms, 1 to 4 heteroatoms selected from an oxygen atom, a sulfur atom and a nitrogen atom, and 1 or 2 rings selected from a 5- or 6-membered aromatic heterocycle containing 1 or 2 nitrogen atoms, a 5-membered aromatic heterocycle containing one sulfur atom and a benzene ring are condensed, and the like.

Examples of the "fused non-aromatic heterocycle" include dihydroindole (e.g., 1,2-dihydroindole), tetrahydroindole (e.g., 4,5,6,7-tetrahydro-1H-indole), dihydroisoindole, tetrahydroisoindole (e.g., 4,5,6,7-tetrahydroisoindole), dihydrobenzofuran, dihydrobenzodioxine (e.g., 2,3-dihydro-1,4-benzodioxine), dihydrobenzodioxepine (e.g., 3,4-dihydro-2H-1,5-benzodioxepine), tetrahydrobenzimidazole (e.g., 4,5,6,7-tetrahydro-1H-benzimidazole), tetrahydrobenzofuran (e.g., 4,5,6,7-tetrahydrobenzofuran), chromene (e.g., 4H-chromene, 2H-chromene), dihydroquinoline (e.g., 1,2-dihydroquinoline), tetrahydroquinoline (e.g., 1,2,3,4-tetrahydroquinoline), dihydroisoquinoline (e.g., 1,2-dihydroisoquinoline), tetrahydroisoquinoline (e.g., 1,2,3,4-tetrahydroisoquinoline), dihydrophthalazine (e.g., 1,4-dihydrophthalazine), 1,4-dihydrocyclopenta[b]pyrrole, 1,4-dihydrocyclopentaimidazole, 1,4-dihydropyrrolo[2,3-d]imidazole and the like.

Examples of the "5- to 7-membered nitrogen-containing heterocycle" in the present specification include pyrrolidine, piperidine and homopiperidine.

While the "compound showing deliquescence at a relative humidity of not less than 80% at 25°C" is not particularly limited as long as it shows deliquescence at a relative humidity of not less than 80% at 25°C. Examples thereof include a low-molecular nonpeptidic compound having a molecular weight of not more than about 1000, preferably not more than about 500, and showing deliquescence at a relative humidity of not less than 80% at 25°C.

While the "compound showing deliquescence at a relative humidity of not less than 80% at 25°C" is preferably "a compound showing deliquescence at a relative humidity of 80% - 90% at 25°C" and is not particularly limited as long as it shows deliquescence at a relative humidity of 80% - 90% at 25°C. Examples thereof include a low-molecular nonpeptidic compound having a molecular weight of not more than about 1000, preferably not more than about 500, and showing deliquescence at a relative humidity of 80% - 90% at 25°C.

Preferred as the "compound showing deliquescence at a relative humidity of not less than 80% at 25°C" is a low-molecular nonpeptidic compound having a secondary amine in the structure and a molecular weight of not more than about 1000, preferably not more than about 500.

More preferred as the "compound showing deliquescence at a relative humidity of not less than 80% at 25°C" is 1-(4-methoxybutyl)-N-(2-methylpropyl)-N-[(3S,5R)-5-(morpholin-4-ylcarbonyl)piperidin-3-yl]-1H-benzimidazole-2-carboxamide (compound (A)) or a salt thereof.

Other preferable examples of the "compound showing deliquescence at a relative humidity of not less than 80% at 25°C" include
a compound represented by the formula (I): 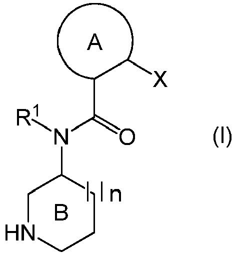 wherein
R¹ is a hydrogen atom, an alkyl group optionally having substituent(s), an alkenyl group optionally having substituent(s), or a cycloalkyl group optionally having substituent(s);
X is absent, or a hydrogen atom, an alkyl group optionally having substituent(s) or a cycloalkyl group optionally having substituent(s);
ring A is heterocycle optionally having substituent(s); ring B is a 5- to 7-membered, nitrogen-containing heterocycle optionally having substituent(s); and
n is 0, 1 or 2,
or a salt thereof.

Each symbol in the formula (I) is defined in detail in the following.

### R¹

In the formula (I), R¹ is a hydrogen atom, an alkyl group optionally having substituent(s), an alkenyl group optionally having substituent(s) or a cycloalkyl group optionally having substituent(s).

Examples of the substituent of the "alkyl group optionally having substituent(s)", "alkenyl group optionally having substituent(s)" and "cycloalkyl group optionally having substituent(s)" for R¹ each include 1 to 5, preferably 1 to 3, substituents selected from the aforementioned substituent group A. When the number of the substituents is two or more, the respective substituents may be the same or different.

R¹ is preferably a C₁₋₆ alkyl group (e.g., isobutyl).

### X

In the formula (I), X is absent or a hydrogen atom, an alkyl group optionally having substituent(s) or a cycloalkyl group optionally having substituent(s).

The "alkyl group" of the "alkyl group optionally having substituent(s)" for X optionally has (e.g., 1 to 5, preferably 1 to 3) substituent(s) at substitutable position(s). When the number of the substituents is two or more, the respective substituents may be the same or different. Examples of the substituent include a halogen atom, a hydroxy group, an alkenyl group optionally having substituent(s), an alkynyl group optionally having substituent(s), a cycloalkyl group optionally having substituent(s), a cycloalkyloxy group optionally having substituent(s), an alkylthio group optionally having substituent(s), an alkylsulfinyl group optionally having substituent(s), an alkylsulfonyl group optionally having substituent(s), an alkoxy group optionally having substituent(s), an aryl group optionally having substituent(s), a heteroaryl group optionally having substituent(s), an aryloxy group optionally having substituent(s), and an acyl group.

Examples of the substituent of the "alkenyl group optionally having substituent(s)", "alkynyl group optionally having substituent(s)", "cycloalkyl group optionally having substituent(s)", "cycloalkyloxy group optionally having substituent(s)", "alkylthio group optionally having substituent(s)", "alkylsulfinyl group optionally having substituent(s)", "alkylsulfonyl group optionally having substituent(s)", "alkoxy group optionally having substituent(s)", "aryl group optionally having substituent(s)", "heteroaryl group optionally having substituent(s)" and "aryloxy group optionally having substituent(s)", which are exemplified as the "substituent" of the "alkyl group optionally having substituent(s)" for X, each include 1 to 5, preferably 1 to 3, substituents selected from the aforementioned substituent group A. When the number of the substituents is two or more, the respective substituents may be the same or different.

Examples of the substituent of the "cycloalkyl group optionally having substituent(s)" for X include 1 to 5, preferably 1 to 3, substituent selected from the aforementioned substituent group A. When the number of the substituents is two or more, the respective substituents may be the same or different.

X is preferably
(1) a hydrogen atom;
(2) a C₁₋₆ alkyl group (e.g., methyl, ethyl, propyl, isopropyl, butyl, hexyl) optionally substituted by substituent(s) selected from
   (a) a halogen atom (e.g., fluorine atom),
   (b) a hydroxy group,
   (c) a C₁₋₆ alkoxy group (e.g., methoxy, ethoxy) optionally having a C₁₋₆ alkoxy group (e.g., methoxy) or a halogen atom (e.g., fluorine atom),
   (d) a C₁₋₆ alkylthio group (e.g., methylthio),
   (e) an aryl group (e.g., phenyl),
   (f) a aryloxy group (e.g., phenyloxy) optionally having a C₁₋₆ alkoxy group (e.g., methoxy) or a halogen atom (e.g., fluorine atom), and
   (g) a heteroaryl group (e.g., thienyl, thiazolyl); or
(3) a C₃₋₁₀ cycloalkyl group (e.g., cyclopropyl), more preferably, a C₁₋₆ alkyl group (e.g., methyl, ethyl, propyl, isopropyl, butyl) optionally substituted by a C₁₋₆ alkoxy group (e.g., methoxy, ethoxy). Preferable examples of the C₁₋₆ alkyl group optionally substituted by a C₁₋₆ alkoxy group include 4-methoxybutyl.

### Ring A

In the formula (I), ring A is a heterocycle optionally having substituent(s).

Ring A is preferably

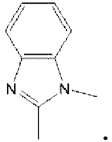

### Ring B

In the formula (I), ring B is a 5- to 7-membered nitrogen-containing heterocycle optionally having substituent(s), n is 0, 1 or 2 and NH constituting ring B is unsubstituted.

Examples of the substituent of the "5- to 7-membered nitrogen-containing heterocycle optionally having substituent(s)" for ring B include 1 to 5, preferably 1 to 3, selected from the aforementioned substituent group A or two substituents bonded to the carbon atoms adjacent to ring B may be bonded to form C₃₋₁₀ cycloalkane (e.g., cyclopentane, cyclohexane) to be condensed with ring B. When the number of the substituents is two or more, the respective substituents may be the same or different.

Ring B is preferably 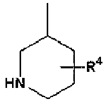 wherein R⁴ is
(1) a hydrogen atom,
(2) a cyano (nitrile) group,
(3) a C₁₋₆ alkyl group (e.g., methyl, ethyl, propyl, isopropyl, butyl, isobutyl, pentyl) optionally having 1 to 3 substituents selected from
   (a) a hydroxy group,
   (b) a C₁₋₆ alkoxy group (e.g., methoxy, ethoxy),
   (c) a C₁₋₆ alkyl-carbonyloxy group (e.g., acetyloxy),
   (d) an aromatic heterocyclic group (e.g., pyridyl, pyrazolyl, triazolyl) optionally having 1 to 3 halogen atoms,
   (e) a C₃₋₁₀ cycloalkyl group (e.g., cyclopropyl), and
   (f) a cyclic amino group (e.g., pyrrolidinyl, piperidino, morpholino, thiomorpholino, piperazinyl, imidazolidin-1-yl, pyrazolidin-1-yl etc.) optionally having an oxo group,
(4) a 3- to 10-membered heterocyclic group (1,2,4-triazolyl, 1,3,4-oxadiazolyl, 1,2,4-oxadiazolyl, 4,5-dihydro-1,2,4-oxadiazolyl, tetrazolyl, tetrahydropyrimidinyl, oxazolyl, piperidinyl, pyrrolidinyl, hexahydropyrimidinyl) optionally having 1 to 3 substituents selected from a C₁₋₆ alkyl group and an oxo group,
(5) a carboxy group,
(6) a C₁₋₆ alkoxy-carbonyl group (e.g., methoxycarbonyl, ethoxycarbonyl, propoxycarbonyl, tert-butoxycarbonyl) optionally having 1 to 3 substituents selected from a nonaromatic heterocyclic group (e.g., dioxolyl) optionally having 1 to 3 substituents selected from a C₁₋₆ alkyl group (e.g., methyl) and oxo group, or
(7) a group represented by the formula: -CO-NR'R"
wherein R' and R" are each a hydrogen atom, or form, together with the nitrogen atom bonded thereto, a nitrogen-containing heterocycle (e.g., azetidine, morpholine, pyrrolidine, piperidine, 7-aza-bicyclo[2.2.1]heptane, homomorpholine, dihydrobenzoxazine (e.g., 3,4-dihydro-2H-1,4-benzoxazine)) optionally having 1 to 3 substituents selected from halogen atom(s) (e.g., fluorine atom).

More preferable ring B is a ring represented by 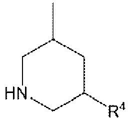 wherein R⁴ is as defined above.

R⁴ is preferably a group represented by the formula: -CO-NR'R" wherein R' and R" are as defined above, more preferably morpholin-4-ylcarbonyl.

Further preferable examples of compound (I) or a salt thereof include the following.

### [Compound I]

A compound represented by the formula (I) wherein ring A is a ring represented by the formula 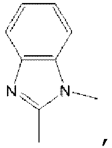
ring B is a ring represented by 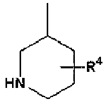
wherein R⁴ is
   (1) a hydrogen atom,
   (2) a cyano (nitrile) group,
   (3) a C₁₋₆ alkyl group (e.g., methyl, ethyl, propyl, isopropyl, butyl, isobutyl, pentyl) optionally having 1 to 3 substituents selected from
      (a) a hydroxy group,
      (b) a C₁₋₆ alkoxy group (e.g., methoxy, ethoxy),
      (c) a C₁₋₆ alkyl-carbonyloxy group (e.g., acetyloxy),
      (d) an aromatic heterocyclic group (e.g., pyridyl, pyrazolyl, triazolyl) optionally having 1 to 3 halogen atoms,
      (e) a C₃₋₁₀ cycloalkyl group (e.g., cyclopropyl), and
      (f) a cyclic amino group (e.g., pyrrolidinyl, piperidino, morpholino, thiomorpholino, piperazinyl, imidazolidin-1-yl, pyrazolidin-1-yl etc.) optionally having an oxo group,
   (4) a 3- to 10-membered heterocyclic group (1,2,4-triazolyl, 1,3,4-oxadiazolyl, 1,2,4-oxadiazolyl, 4,5-dihydro-1,2,4-oxadiazolyl, tetrazolyl, tetrahydropyrimidinyl, oxazolyl, piperidinyl, pyrrolidinyl, hexahydropyrimidinyl) optionally having 1 to 3 substituents selected from a C₁₋₆ alkyl group and an oxo group,
   (5) a carboxy group,
   (6) a C₁₋₆ alkoxy-carbonyl group (e.g., methoxycarbonyl, ethoxycarbonyl, propoxycarbonyl, tert-butoxycarbonyl) optionally having 1 to 3 substituents selected from a nonaromatic heterocyclic group (e.g., dioxolyl) optionally having 1 to 3 substituents selected from a C₁₋₆ alkyl group (e.g., methyl) and oxo group, or
   (7) a group represented by the formula: -CO-NR'R" wherein R' and R" are each a hydrogen atom, or
R' and R" form, together with the nitrogen atom bonded thereto, a nitrogen-containing heterocycle (e.g., azetidine, morpholine, pyrrolidine, piperidine, 7-aza-bicyclo[2.2.1]heptane, homomorpholine, dihydrobenzoxazin (e.g., 3,4-dihydro-2H-1,4-benzoxazin)) optionally having 1 to 3 substituents selected from halogen atom(s) (e.g., fluorine atom),
R¹ is a C₁₋₆ alkyl group (e.g., isobutyl), and
X is
   (1) a hydrogen atom;
   (2) a C₁₋₆ alkyl group (e.g., methyl, ethyl, propyl, isopropyl, butyl, hexyl) optionally substituted by substituent(s) selected from
      (a) a halogen atom (e.g., fluorine atom),
      (b) a hydroxy group,
      (c) a C₁₋₆ alkoxy group (e.g., methoxy, ethoxy) optionally having a C₁₋₆ alkoxy group (e.g., methoxy) or a halogen atom (e.g., fluorine atom),
      (d) a C₁₋₆ alkylthio group (e.g., methylthio),
      (e) an aryl group (e.g., phenyl),
      (f) an aryloxy group (e.g., phenyloxy) optionally having a C₁₋₆ alkoxy group (e.g., methoxy) or a halogen atom (e.g., fluorine atom), and
      (g) a heteroaryl group (e.g., thienyl, thiazolyl); or
   (3) a C₃₋₁₀ cycloalkyl group (e.g., cyclopropyl),
      or a salt thereof.

Preferable compound (I) or a salt thereof is compound (A) or a salt thereof.

Compound (I) or a salt thereof can be produced according to a method known per se, for example, the production method described in WO2009/154300 or a method analogous thereto.

Examples of the salts of compound (I) and compound (A) include metal salts, ammonium salts, salts with organic bases, salts with inorganic acids, salts with organic acids, salts with basic or acidic amino acids, and the like.

Preferable examples of the metal salt include alkali metal salts such as sodium salt, potassium salt and the like; alkaline earth metal salts such as calcium salt, magnesium salt, barium salt and the like; aluminum salt and the like.

Preferable examples of the salt with organic base include a salt with trimethylamine, triethylamine, pyridine, picoline, 2,6-lutidine, ethanolamine, diethanolamine, triethanolamine, cyclohexylamine, dicyclohexylamine, N,N'-dibenzylethylenediamine or the like.

Preferable examples of the salt with inorganic acid include a salt with hydrochloric acid (e.g., monohydrochloride, dihydrochloride), hydrobromic acid, nitric acid, sulfuric acid, phosphoric acid or the like.

Preferable examples of the salt with organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid or the like.

Preferable examples of the salt with basic amino acid include a salt with arginine, lysine, ornithine or the like.

Preferable examples of the salt with acidic amino acid include a salt with aspartic acid, glutamic acid or the like.

Preferable compound (A) is hydrochloride, and monohydrochloride is particularly preferable.

In the tablet of the present specification, the "compound showing deliquescence at a relative humidity of not less than 80% at 25°C" is used at a content of preferably about 1 - about 40 wt%, more preferably about 1.5 - about 25 wt%, of one tablet of the present specification.

In one preferable embodiment of the tablet of the present specification wherein compound (I) (particularly, compound (A)) or a salt thereof is used as the "compound showing deliquescence at a relative humidity of not less than 80% at 25°C", compound (I) (particularly compound (A)) or a salt thereof is used at a content of preferably about 1 - about 40 wt%, more preferably about 1.5 - about 25 wt%, as compound (I) (particularly compound (A)), of one tablet of the present specification.

In another preferable embodiment of the tablet of the present specification wherein compound (A) or a salt thereof is used as the "compound showing deliquescence at a relative humidity of not less than 80% at 25°C", compound (A) or a salt thereof is used at a content of preferably about 3 - about 40 wt%, more preferably about 3 - about 20 wt%, as compound (A), of one tablet of the present specification.

Examples of the "additive showing an equilibrium moisture absorption rate at relative humidity 75% at 25°C of not less than 7 wt% relative to its dry weight" to be used in the present specification include crystalline cellulose, low-substituted hydroxypropylcellulose, hydroxypropylcellulose, hydroxypropylmethylcellulose, croscarmellose sodium, carmellose calcium and the like.

When the tablet of the present specification is a core tablet or a film-coated tablet, the "additive showing an equilibrium moisture absorption rate at relative humidity 75% at 25°C of not less than 7 wt% relative to its dry weight" may be contained in any of the film coating layer and the core tablet. For example, hydroxypropylcellulose and hydroxypropylmethylcellulose (e.g., Hypromellose 2910 (Shin-Etsu Chemical Co., Ltd.)) may be used as a binder, a disintegrant, a water-soluble film coating base and the like in amounts within the contents to be described later.

The "additive showing an equilibrium moisture absorption rate at relative humidity 75% at 25°C of not less than 7 wt% relative to its dry weight" to be used in the present specification may be a combination of one or more kinds of the above-mentioned additives.

In the tablet of the present specification, one or more kinds of the "additive showing an equilibrium moisture absorption rate at relative humidity 75% at 25°C of not less than 7 wt% relative to its dry weight" is(are) used at a (total) content of 35 wt% or more of one tablet of the present specification.

While the crystalline cellulose to be used in the present invention is not particularly limited as long as it is used as an additive for pharmaceutical products, crystalline cellulose, crystalline cellulose (granule), crystalline cellulose (fine particle) and the like may be used alone or two or more kinds thereof may be mixed and used.

Specific examples of the crystalline cellulose include CEOLUS PH-101 (Asahi Kasei Chemicals Corporation), CEOLUS PH-302 (Asahi Kasei Chemicals Corporation), CEOLUS KG-802 (Asahi Kasei Chemicals Corporation) and the like.

In the tablet of the present specification, when crystalline cellulose alone is used as the "additive showing an equilibrium moisture absorption rate at relative humidity 75% at 25°C of not less than 7 wt% relative to its dry weight", it is used at a content of not less than about 35 wt% of one tablet of the present specification. When crystalline cellulose is used in combination with other additive mentioned above, it is used at a content of not less than about 30 wt%, preferably about 30 - about 50 wt%, more preferably about 30 - about 40 wt%, of one tablet of the present specification.

The low-substituted hydroxypropylcellulose to be used in the present invention is not particularly limited as long as it is used as a pharmaceutical additive. It may be used alone or two or more kinds thereof may be mixed and used.

In the present specification, low substitution rate means that 5.0% - 16.0% of a hydroxypropoxyl group is contained after drying.

Specific examples of the low-substituted hydroxypropylcellulose include LH-11 (Shin-Etsu Chemical Co., Ltd.), LH-21 (Shin-Etsu Chemical Co., Ltd.), LH-22 (Shin-Etsu Chemical Co., Ltd.) and the like.

In the tablet of the present specification, when low-substituted hydroxypropylcellulose alone is used as the "additive showing an equilibrium moisture absorption rate at relative humidity 75% at 25°C of not less than 7 wt% relative to its dry weight", it is used at a content of not less than about 35 wt% of one tablet of the present specification. When low-substituted hydroxypropylcellulose is used in combination with other additive mentioned above, it is used at a content of not less than about 5 wt%, preferably about 5 - about 20 wt%, more preferably about 7 - about 9 wt%, of one tablet of the present specification.

The "additive showing an equilibrium moisture absorption rate at relative humidity 75% at 25°C of not less than 7 wt% relative to its dry weight" is preferably crystalline cellulose.

In the tablet of the present specification, when crystalline cellulose is used as the "additive showing an equilibrium moisture absorption rate at relative humidity 75% at 25°C of not less than 7 wt% relative to its dry weight", crystalline cellulose is preferably used at a content of not less than about 30 wt%, of one tablet of the present specification. More preferably, crystalline cellulose is used at a content of about 30 - about 50 wt%, of one tablet of the present specification. Further preferably, crystalline cellulose is used at a content of about 30 - about 40 wt%, of one tablet of the present specification.

The "additive showing an equilibrium moisture absorption rate at relative humidity 75% at 25°C of not less than 7 wt% relative to its dry weight" is preferably low-substituted hydroxypropylcellulose.

In the tablet of the present specification, when low-substituted hydroxypropylcellulose is used as the "additive showing an equilibrium moisture absorption rate at relative humidity 75% at 25°C of not less than 7 wt% relative to its dry weight", low-substituted hydroxypropylcellulose is used at a content of not less than about 5 wt%, of one tablet of the present specification. More preferably, low-substituted hydroxypropylcellulose is used at a content of about 5 - about 20 wt%, of one tablet of the present specification. Further preferably, low-substituted hydroxypropylcellulose is used at a content of about 7 - about 9 wt%, of one tablet of the present specification.

The "additive showing an equilibrium moisture absorption rate at relative humidity 75% at 25°C of not less than 7 wt% relative to its dry weight" is more preferably crystalline cellulose and low-substituted hydroxypropylcellulose used in combination.

In the tablet of the present specification, when crystalline cellulose and low-substituted hydroxypropylcellulose are used as the "additive showing an equilibrium moisture absorption rate at relative humidity 75% at 25°C of not less than 7 wt% relative to its dry weight", crystalline cellulose is preferably used at a content of not less than about 30 wt% and low-substituted hydroxypropylcellulose is used at a content of not less than about 5 wt%, each of one tablet of the present invention. In the tablet of the present invention, crystalline cellulose is used at a content of 30 - 50 wt% and low-substituted hydroxypropylcellulose is used at a content of 5 - 20 wt%, each of one tablet of the present invention. Preferably, crystalline cellulose is used at a content of about 30 - about 40 wt% and low-substituted hydroxypropylcellulose is used at a content of about 7 - about 9 wt%, each of one tablet of the present specification.

The tablet of the present invention may further contain an additive conventionally used in the field of pharmaceutical preparation. Examples of the additive include excipients, binder, disintegrant, colorant, pH adjuster, surfactant, stabilizer, acidulant, flavor, fluidizer, lubricant, coating base, coating additive and the like. Unless particularly indicated, these additives are used in an amount conventionally employed in the field of pharmaceutical preparation.

Preferable examples of the excipient include mannitol; starches such as corn starch, potato starch, wheat starch, rice starch, partly pregelatinized starch, pregelatinized starch, perforated starch and the like; anhydrous calcium phosphate; precipitated calcium carbonate; calcium silicate and the like. Among these, mannitol is preferable.

In the tablet of the present specification, an excipient is used at a content of preferably about 20 - about 60 wt%, more preferably about 30 - about 50 wt%, of one tablet of the present specification.

Preferable examples of the binder include hydroxypropylcellulose, hydroxypropylmethylcellulose, polyvinylpyrrolidone (povidone), gum arabic and the like.

Preferable examples of the disintegrant include hydroxypropylcellulose, carboxymethylcellulose, carboxymethylcellulose calcium, sodium carboxymethyl starch, croscarmellose sodium, croscarmellose calcium, crospovidone, hydroxypropylstarch and the like.

Preferable examples of the colorant include food colors such as Food Yellow No. 5, Food Red No. 2, Food Blue No. 2 and the like, food lake colors, red ferric oxide, yellow ferric oxide, titanium oxide and the like.

Preferable examples of the pH adjuster include citrate, phosphate, carbonate, tartrate, fumarate, acetate, amino acid salt and the like.

Preferable examples of the surfactant include sodium lauryl sulfate, polysorbate 80, polyoxyethylene(160)polyoxypropylene(30)glycol and the like.

Preferable examples of the stabilizer include tocopherol, tetrasodium edetate, nicotinic acid amide, cyclodextrins and the like.

Preferable examples of the acidulant include ascorbic acid, citric acid, tartaric acid, malic acid and the like.

Preferable examples of the flavor include menthol, peppermint oil, lemon oil, vanillin and the like.

Preferable examples of the fluidizer include light anhydrous silicic acid, hydrated silicon dioxide, talc and the like. Among these, light anhydrous silicic acid is preferable.

The fluidizer is used at a content of preferably about 0.1 - about 1 wt%, more preferably about 0.2 - about 0.5 wt%, of one tablet of the present invention.

Preferable examples of the lubricant include magnesium stearate, calcium stearate, talc, sucrose esters of fatty acids, sodium stearyl fumarate and the like. Among these, magnesium stearate is preferable.

The lubricant is used at a content of preferably about 0.1 - about 5 wt%, more preferably about 1 - about 3 wt%, of one tablet of the present invention.

Preferable examples of the coating base include sugar coating base, aqueous film coating base, enteric film coating base, sustained-release film coating base and the like.

As the sugar coating base, sucrose can be used. Furthermore, one or more kinds selected from talc, precipitated calcium carbonate, gelatin, gum arabic, pullulan, carnauba wax and the like may be used in combination.

Examples of the aqueous film coating base include cellulose polymers such as hydroxypropylcellulose, hydroxypropylmethylcellulose (e.g., Hypromellose 2910 (Shin-Etsu Chemical Co., Ltd.), hydroxyethylcellulose, methylhydroxyethylcellulose and the like; synthetic polymers such as polyvinyl acetal diethylaminoacetate, aminoalkylmethacrylate copolymer E (e.g., Eudragit E (trade name)), polyvinylpyrrolidone and the like; polysaccharides such as pullulan and the like, and the like.

Examples of the enteric film coating base include cellulose polymers such as hydroxypropylmethylcellulose phthalate, hydroxypropylmethylcellulose acetate succinate, carboxymethylethylcellulose, cellulose acetate phthalate and the like; acrylic acid polymers such as methacrylic acid copolymer L (e.g., Eudragit L (trade name)), methacrylic acid copolymer LD (e.g., Eudragit L-30D55 (trade name)), methacrylic acid copolymer S (e.g., Eudragit S (trade name)) and the like; naturally occurring substances such as shellac and the like; and the like.

Examples of the sustained-release film coating base include cellulose polymers such as ethylcellulose and the like; acrylic acid polymers such as aminoalkyl methacrylate copolymer RS (e.g., Eudragit RS (trade name)), ethyl acrylate-methyl methacrylate copolymer suspension (e.g., Eudragit NE (trade name)) and the like; and the like.

Preferable examples of the coating additive include light shielding agents such as titanium dioxide and the like, fluidizers such as talc and the like; colorants such as red ferric oxide, yellow ferric oxide and the like; plasticizers such as polyethylene glycol (e.g., macrogol 6000), triethyl citrate, castor oil, polysorbates and the like; organic acids such as citric acid, tartaric acid, malic acid, ascorbic acid and the like; and the like.

Two or more kinds of the above-mentioned additives may be mixed at an appropriate ratio and used.

Specific preferable examples of the tablet of the present specification or the tablet of the present invention include the following:
(tablet A: according to the specification)
   A tablet containing the following (i) - (iii):
   (i) compound (A) (preferably hydrochloride of compound (A), more preferably monohydrochloride of compound (A)),
   (ii) not less than 30 wt% of crystalline cellulose in the tablet (of the weight of one tablet), and
   (iii) not less than 5 wt% of low-substituted hydroxypropylcellulose in the tablet (of the weight of one tablet).
(tablet B: according to the invention)
   A tablet containing the following (i) - (iii):
   (i) compound (A) (preferably hydrochloride of compound (A), more preferably monohydrochloride of compound (A)),
   (ii) 30 - 50 wt% of crystalline cellulose in the tablet (of the weight of one tablet), and
   (iii) 5 - 20 wt% of low-substituted hydroxypropylcellulose in the tablet (of the weight of one tablet).
(tablet C: according to the invention)
   A tablet containing the following (i) - (iii):
   (i) 1.5 - 25 wt% of compound (A) (preferably hydrochloride of compound (A), more preferably monohydrochloride of compound (A)), (of the weight of one tablet),
   (ii) 30 - 50 wt% of crystalline cellulose in the tablet (of the weight of one tablet), and
   (iii) 5 - 20 wt% of low-substituted hydroxypropylcellulose in the tablet (of the weight of one tablet).
(tablet D: according to the invention)
   A tablet containing the following (i) - (iii):
   (i) 1.5 - 25 wt% of compound (A) (preferably hydrochloride of compound (A), more preferably monohydrochloride of compound (A)), (of the weight of one tablet),
   (ii) 30 - 40 wt% of crystalline cellulose in the tablet (of the weight of one tablet), and
   (iii) 7 - 9 wt% of low-substituted hydroxypropylcellulose in the tablet (of the weight of one tablet).

The tablet of the present invention may be film-coated from the aspects of dosability, strength of preparation and the like. Preferable examples of the coating base and coating additive used for film coating include those similar to the coating base and coating additive used for the aforementioned tablet of the present invention.

When the tablet of the present invention is film-coated, a film coating layer is generally formed in the proportion of 1 - 10 parts by weight, preferably 2 - 6 parts by weight, per 100 parts by weight of the tablet of the present invention (core tablet) before coating.

The tablet of the present specification can be produced by granulating and mixing compound (I) or a salt thereof, crystalline cellulose, low-substituted hydroxypropylcellulose and, where necessary, other additive, and tableting the mixture.

In the production process of the tablet of the present invention, operations of granulation, mixing, tableting, coating and the like can be performed by a method conventionally used in the formulation technical field.

Granulation is performed using, for example, a granulator such as high speed mixer granulator, fluid bed dryer granulator, extrusion granulator, roller compacter and the like. Mixing is performed using, for example, a mixer such as V-type mixer, tumbler mixer and the like.

Tableting (punching) is performed using, for example, a single punch tableting machine, a rotary tableting machine and the like.

When a single punch tableting machine, a rotary tableting machine or the like is used, generally, a tableting pressure of 1 - 45 kN/cm² (preferably 5 - 40 kN/cm²) is preferably adopted, and further, a tapered die is preferably used for the purpose of preventing capping.

The coating is performed by, for example, using a film coating apparatus and the like.

The tablet of the present invention preferably has a hardness of about 50 - about 200 N.

The tablet of the present invention may be film-coated from the aspects of dosability, strength of preparation and the like. In addition, the above-mentioned tablet may be filled in a capsule (e.g., gelatin capsule) to give a capsule.

In addition, the tablet of the present invention may be printed with stamps or letters for discrimination, or have a separating line for dividing the tablet.

The tablet of the present invention can be safely administered orally to a mammal (e.g., mouse, rat, rabbit, cat, dog, bovine, horse, monkey, human).

In the present specification, a tablet containing compound (I) as a pharmaceutically active ingredient is useful as a safe prophylactic or therapeutic agent for various diseases caused by the renin-angiotensin system (RA system).

Examples of the various diseases caused by RA system include hypertension (e.g., essential hypertension, renovascular hypertension, renal parenchymal hypertension, primary aldosteronism, Cushing's syndrome), circadian blood pressure abnormality, cardiac disease (e.g., cardiac hypertrophy, acute cardiac failure, chronic cardiac failure including congestion, diastolic heart failure, cardiomyopathy, angina pectoris, myocarditis, atrial fibrillation, arrhythmia, tachycardia, myocardial infarction), cerebrovascular disorder (e.g., asymptomatic cerebrovascular disorder, transient cerebral ischemic attack, cerebrovascular dementia, hypertensive encephalopathy, cerebral infarction), brain edema, brain circulation disorder, recurrence and sequelae of cerebrovascular disorder (e.g., neural symptoms, mental symptoms, subjective symptoms, activities of daily living impairment), ischemic peripheral circulation disorder, myocardial ischemia, venous insufficiency, cardiac failure progress after myocardial infarction, renal diseases (e.g., nephritis, glomerulonephritis, glomerulosclerosis, renal failure, nephrosis syndrome, thrombotic microangiopathy, dialysis complications, organ disorder including nephropathy due to radiation exposure), arteriosclerosis including atherosclerosis (e.g., aneurysm, coronary sclerosis, cerebral arterial sclerosis, peripheral arterial sclerosis), vascular hypertrophy, vascular hypertrophy or occlusion and organ disorder after intervention (e.g., percutaneous transluminal coronary angioplasty, stenting, coronary angioscopy, intravascular ultrasound, intracoronary thrombolytic therapy), blood vessel reocclusion·restenosis after bypass surgery, polycythemia·hypertension·organ disorder·vascular hypertrophy of post-transplantation, rejection of post-transplantation, ophthalmic diseases (e.g., glaucoma, ocular hypertension), thrombosis, multiple organ failure, endothelial dysfunction, hypertensive tinnitus, the other circulatory diseases (e.g., deep vein thrombosis, obstructive peripheral circulation disorder, arteriosclerosis obliterans, thromboangiitis obliterans, ischemic cerebral circulatory disorder, Raynaud's disease, Buerger's disease), metabolism·malnutrition (e.g., diabetes, impaired glucose tolerance, insulin resistance, hyperinsulinemia, diabetic nephropathy, diabetic retinopathy, diabetic neuropathy, obesity, hyperlipidemia, hypercholesterolemia, hyperuricemia, hyperkalemia, hypernatremia), metabolic syndrome, nonalcoholic steatohepatitis (NASH), nonalcoholic fatty liver diseases (NAFLD), neurodegenerative disease (e.g., Alzheimer's disease, Parkinson's disease, Creutzfeldt-Jakob disease, multiple sclerosis, amyotrophic lateral sclerosis, AIDS encephalopathy), central nervous disorders (e.g., disorders such as cerebral hemorrhage, cerebral infarction and the like, and sequelae· complications thereof, head trauma, spinal injury, brain edema, disorders of sensory function, abnormality of sensory function, disorders of autonomic nervous function, abnormality of autonomic nervous function), dementia, migraine, memory disorders, disturbance of consciousness, amnesia, anxiety, tension symptom, anxious mental state, sleep disorder, insomnia, mental diseases (e.g., depression, epilepsy, alcohol dependence), inflammatory disease (e.g., arthritis such as rheumatoid arthritis, osteoarthritis, rheumatoid myelitis, periostitis and the like; inflammation after surgery·trauma; regression of puffiness; pharyngitis; bladder inflammation; pneumonia; atopic dermatitis; inflammatory bowel disease such as Crohn's disease, ulcerative colitis and the like; meningitis; inflammatory ocular disease; inflammatory pulmonary diseases such as pneumonia, silicosis, lung sarcoidosis, pulmonary tuberculosis and the like), allergic disease (e.g., allergic rhinitis, conjunctivitis, gastrointestinal allergy, pollinosis, anaphylaxis), chronic obstructive pulmonary diseases, interstitial pneumonia, carinii pneumonia, collagen disease (e.g., systemic lupus erythematosus, scleroderma, polyarteritis), liver disease (e.g., hepatitis including chronic stage, cirrhosis), portal hypertension, digestive tract diseases (e.g., gastritis, gastric ulcer, gastric cancer, postgastrostomy disturbances, dyspepsia, esophageal ulcer, pancreatitis, colon polyp, cholelithiasis, hemorrhoids, variceal rupture of esophagus and stomach), blood·hematopoietic organ disease (e.g., polycythemia, vascular peliosis, autoimmune hemolytic anemia, disseminated intravascular coagulation syndrome, multiple myelosis), bone disease (e.g., bone fracture, bone refracture, osteoporosis, osteomalacia, bone Paget's disease, rigid myelitis, rheumatoid arthritis, knee osteoarthritis and destruction of articular tissue in disease similar thereto), solid tumor, tumor (e.g., malignant melanoma, malignant lymphoma, gastrointestinal (e.g., stomach, bowels) cancer), cancer and cachexia associated therewith, cancer metastasis, endocrine diseases (e.g., Addison's disease, pheochromocytoma), urinary organs·male genital disease (e.g., bladder inflammation, prostatomegaly, prostate cancer, sexually-transmitted diseases), gynecologic diseases (e.g., menopausal disorder, gestational toxicosis, endometriosis, hysteromyoma, ovarian disease, mammary gland disease, sexually-transmitted diseases), disease due to environment·occupational factor (e.g., radiation disorder, disorder due to UV·infrared· laser beam, altitude sickness), respiratory diseases (e.g., cold syndrome, pneumonia, asthma, pulmonary hypertension, pulmonary thrombus·pulmonary embolism), infections (e.g., virus infections such as cytomegalovirus, influenza virus, herpes virus and the like, rickettsial infections, bacterium infections), toxemia (e.g., sepsis, septic shock, endotoxin shock, gram negative sepsis, toxic shock syndrome), otorhinolaryngologic diseases (e.g., Meniere syndrome, tinnitus, gustation disorder, dizziness, dysequilibrium, dysphagia), dermatic diseases (e.g., keloid, Hemangioma, psoriasis), ophthalmic diseases (e.g., cataract, glaucoma), systemic disease such as dialysis hypotension, myasthenia gravis, chronic fatigue syndrome etc. and the like.

The various diseases caused by RA system also include circulatory diseases, various organ disorders caused by hypertension and the like.

The circulatory diseases include, for example, hypertension, circadian blood pressure abnormality, cardiac disease, cerebrovascular disorder, brain edema, brain circulation disorder, recurrence and sequelae of cerebrovascular disorder, ischemic peripheral circulation disorder, myocardial ischemia, venous insufficiency, cardiac failure progress after myocardial infarction, renal diseases, arteriosclerosis including atherosclerosis, vascular hypertrophy, vascular hypertrophy or occlusion and organ disorder after intervention, blood vessel reocclusion· restenosis after bypass surgery, post-transplantation hypertension·organ disorder·vascular hypertrophy, thrombosis, multiple organ failure, endothelial dysfunction, hypertensive tinnitus, migraine, blood·hematopoietic organ disease, dialysis hypotension and the like.

The various organ disorders caused by hypertension include cardiac disease, encephalopathy, renal diseases, multiple organ failure and the like.

Specifically, for example, the effective amount of compound (I) as its free form is generally 0.01 - 1000 mg/day, preferably 1 - 500 mg/day, more preferably 5 - 250 mg/day, further preferably 5 - 100 mg/day, per an adult (body weight 60 kg). The administration frequency of the tablet, containing compound (I) as a pharmaceutically active ingredient, to the aforementioned mammal per day is preferably 1 - 3 times.

Particularly preferable examples of the tablet containing compound (I) as pharmaceutically active ingredient include "a tablet containing 5 mg of compound (A) (free form) per tablet";
"a tablet containing 10 mg of compound (A) (free form) per tablet";
"a tablet containing 20 mg of compound (A) (free form) per tablet";
"a tablet containing 40 mg of compound (A) (free form) per tablet"; and
"a tablet containing 50 mg of compound (A) (free form) per tablet".

The tablet of the present invention can be used in combination with one or more kinds of other drugs (hereinafter sometimes to be abbreviated as concomitant drug).

Specific examples of the combination drug include the following:

### (1) antihypertensive agent

Angiotensin-converting enzyme inhibitor (e.g., captopril, enalapril maleate, alacepril, delapril hydrochloride, imidapril hydrochloride, quinapril hydrochloride, cilazapril, temocapril hydrochloride, trandolapril, benazepril hydrochloride, perindopril, lisinopril, ramipril), angiotensin II antagonist (e.g., candesartan cilexetil, candesartan, losartan, losartan potassium, eprosartan, valsartan, telmisartan, irbesartan, tasosartan, olmesartan, olmesartan medoxomil, azilsartan, azilsartan medoxomil), aldosterone receptor antagonists (spironolactone, eplerenone), calcium antagonist (e.g., verapamil hydrochloride, diltiazem hydrochloride, nifedipine, amlodipine besilate, azelnidipine, aranidipine, efonidipine hydrochloride, cilnidipine, nicardipine hydrochloride, nisoldipine, nitrendipine, nilvadipine, barnidipine hydrochloride, felodipine, benidipine hydrochloride, manidipine hydrochloride), β blocker (e.g., metoprolol tartrate, atenolol, propranolol hydrochloride, bisoprolol fumarate), αβ blocker (e.g., carvedilol), clonidine, diuretic (e.g., theobromine sodium salicylate, theobromine calcium salicylate, ethiazide, cyclopenthiazide, trichlormethiazide, hydrochlorothiazide, hydroflumethiazide, benzylhydrochlorothiazide, penfluthiazide, poly5thiazide, methyclothiazide, acetazolamide, tripamide, meticrane, chlorthialidone, mefruside, indapamide, azosemide, isosorbide, ethacrynic acid, piretanide, bumetanide, furosemide) and the like.

### (2) antithrombotic agent

Anticoagulant (e.g., heparin sodium, heparin calcium, warfarin calcium (e.g., warfarin), anti-thrombin drug (e.g., argatroban, dabigatran), activated blood coagulation factor Xa inhibitor and medicament having function redressing the balance of coagulation fibrinolytic system (e.g., rivaroxaban, apixaban, edoxaban, YM-150, the compound described in WO 02/06234, WO 2004/048363, WO 2005/030740, WO 2005/058823, WO 2005/113504 or WO 2004/048363)), thrombolytic drug (e.g., tPA, urokinase, tisokinase, alteplase, nateplase, monteplase, pamiteplase), antiplatelet drug (e.g., aspirin, sulfinpyrazone (anturane), dipyridamole (persantine), ticlopidine hydrochloride (panaldine), cilostazol (pletal), GPIIb/IIIa antagonist (e.g., ReoPro etc.), clopidogrel, prasugrel, ticagrelor, E5555, SHC530348, ethyl icosapentate, beraprost sodium, sarpogrelate hydrochloride) and the like.

### (3) therapeutic agent for diabetes

Insulin preparation (e.g., animal insulin preparation extracted from pancreas of bovine and swine; human insulin preparation genetic engineering-synthesized using Escherichia coli and yeast; zinc insulin; protamine zinc insulin; insulin fragment or derivative (e.g., INS-1), oral insulin preparation), insulin sensitizer (e.g., pioglitazone or a salt thereof (preferably, hydrochloride), rosiglitazone or a salt thereof (preferably, maleate), Metaglidasen, AMG-131, Balaglitazone, MBX-2044, Rivoglitazone, Aleglitazar, Chiglitazar, Lobeglitazone, PLX-204, PN-2034, GFT-505, THR-0921, the compound described in WO 2007/013694, WO 2007/018314, WO 2008/093639 or WO 2008/099794), α-glucosidase inhibitor (e.g., voglibose, acarbose, miglitol, emiglitate), biguanide (e.g., phenformin, metformin, buformin or a salt thereof (e.g., hydrochloride, fumarate, succinate) etc.), insulin secretagogue (e.g., sulfonylurea (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or a calcium salt hydrate thereof), dipeptidyl peptidase IV inhibitor (e.g., Alogliptin or a salt thereof (preferably, benzoate), Vildagliptin, Sitagliptin, Saxagliptin, BI1356, GRC8200, MP-513, PF-00734200, PHX1149, SK-0403, ALS2-0426, TA-6666, TS-021, KRP-104, 2-[[6-[(3R)-3-amino-1-piperidinyl]-3,4-dihydro-3-methyl-2,4-dioxo-1(2H)-pyrimidinyl]methyl]-4-fluorobenzonitrile or a salt thereof), β3 agonist (e.g., N-5984), GPR40 agonist (e.g., the compound described in WO 2004/041266, WO 2004/106276, WO 2005/063729, WO 2005/063725, WO 2005/087710, WO 2005/095338, WO 2007/013689 or WO 2008/001931), GLP-1 receptor agonist (e.g., GLP-1, GLP-1MR agent, Liraglutide, Exenatide, AVE-0010, BIM-51077, Aib(8,35)hGLP-1(7,37)NH₂, CJC-1131, Albiglutide, amylin agonist (e.g., pramlintide), phosphotyrosine phosphatase inhibitors (e.g., sodium vanadate), gluconeogenesis inhibitor (e.g., glycogen phosphorylase inhibitor, glucose-6-phosphatase inhibitors, glucagon antagonists, FBPase inhibitor etc.), SGLT2 (sodium-glucose cotransporter 2) inhibitor (e.g., Depagliflozin, AVE2268, TS-033, YM543, TA-7284, Remogliflozin, ASP1941), SGLT1 inhibitor, 11β-hydroxy steroid dehydrogenase inhibitor (e.g., BVT-3498, INCB-13739), adiponectin or an agonist thereof, IKK inhibitor (e.g., AS-2868), leptin resistance improving drugs, somatostatin receptor agonists, glucokinase activators (e.g., Piragliatin, AZD1656, AZD6370, TTP-355, the compound described in WO 2006/112549, WO 2007/028135, WO 2008/047821, WO 2008/050821, WO 2008/136428 or WO 2008/156757), GIP (Glucose-dependent insulinotropic peptide), GPR119 agonist (e.g., PSN821), FGF21, FGF analogue and the like.

### (4) therapeutic agents for diabetic complications

Aldose reductase inhibitors (e.g., tolrestat, epalrestat, zopolrestat, fidarestat, CT-112, ranirestat (AS-3201), Lidorestat), neurotrophic factor and an increasing drug thereof (e.g., NGF, NT-3, BDNF, neurotrophic factors and increasing drugs thereof described in WO 01/14372 (e.g., 4-(4-chlorophenyl)-2-(2-methyl-1-imidazolyl)-5-[3-(2-methylphenoxy)propyl]oxazole), the compound described in WO 2004/039365), PKC inhibitor (e.g., ruboxistaurin mesylate), AGE inhibitor (e.g., ALT946, N-phenacylthiazolium bromide (ALT766), EXO-226, Pyridorin, pyridoxamine), GABA receptor agonist (e.g., gabapentin, Pregabalin), serotonin·noradrenaline reuptake inhibitor (e.g., duloxetine), sodium channel inhibitor (e.g., Lacosamide), active oxygen scavengers (e.g., thioctic acid), cerebral vasodilator (e.g., tiapuride, mexiletine), somatostatin receptor agonists (e.g., BIM23190), apoptosis signal regulating kinase-1 (ASK-1) inhibitor and the like.

### (5) antilipidemic agent

HMG-CoA reductase inhibitor (e.g., pravastatin, simvastatin, lovastatin, atorvastatin, fluvastatin, rosuvastatin, pitavastatin or a salt thereof (e.g., sodium salt, calcium salt)), squalene synthase inhibitors (e.g., the compound described in WO 97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compound (e.g., bezafibrate, clofibrate, simfibrate, clinofibrate), anion exchange resin (e.g., colestyramine), probucol, nicotinic acid drugs (e.g., nicomol, niceritrol, niaspan), ethyl icosapentate, phytosterol (e.g., soysterol, γ-oryzanol), cholesterol absorption inhibitor (e.g., Zetia), CETP inhibitor (e.g., dalcetrapib, anacetrapib), ω-3 fatty acid preparation (e.g., ω-3-acid ethyl esters 90) and the like.

Besides the above, they can be used in combination with other pharmaceutical ingredients including therapeutic drugs for bone diseases, myocardial protective drugs, therapeutic drugs for coronary heart diseases, therapeutic drugs for chronic cardiac failure, therapeutic drugs for hypothyroidism, therapeutic drugs for nephrosis syndrome, therapeutic drugs for chronic renal failure, therapeutic agents for renal anemia (e.g., erythropoietin preparation, peginesatide), therapeutic drugs for gynecologic diseases and therapeutic drugs for infections.

Furthermore, the drug to be used in combination may be an antibody drug or a nucleic acid drug.

Moreover, the preparation of the present specification can also be used along with a gene therapy.

When the tablet of the present invention and a concomitant drug are used in combination, the administration time of these is not limited, and they can be administered simultaneously to an administration subject, or may be administered in a staggered manner.

In addition, the tablet of the present invention and the concomitant drug may be administered as separate preparations or as a single preparation containing the tablet of the present invention and the concomitant drug, to an administration subject.

The dose of the concomitant drug can be appropriately determined based on the clinically employed dose of each drug. In addition, the mixing ratio of the tablet of the present invention and the concomitant drug can be appropriately determined according to the administration subject, administration route, target disease, condition, combination and the like. For example, when the administration subject is a human, the concomitant drug may be used in an amount of 0.01 to 100 parts by weight per 1 part by weight of the tablet of the present invention.

Use of the concomitant drug in this way provides superior effects such as 1) enhanced action of the tablet of the present invention or the concomitant drug (synergistic effect of the actions of the medicaments), 2) reduced dose of the tablet of the present invention or the combination drug (effect of reduction of dose of medicaments as compared to single drug administration), 3) reduced secondary action of the tablet of the present invention or the concomitant drug, and the like.

The present invention is explained in more detail in the following by referring to Examples and Experimental Examples.

As additives for pharmaceutical preparations in the following Examples, the Japanese Pharmacopoeia 16th edition, the Japanese Pharmacopoeia Japanese Pharmaceutical Codex or Japanese Pharmaceutical Excipients 2003 compatible products were used.

### Examples

### Example 1

According to the formulation of Table 1, hydrochloride (monohydrochloride) of compound (A), mannitol, crystalline cellulose (1), low-substituted hydroxypropylcellulose, magnesium stearate (1) and light anhydrous silicic acid were uniformly mixed in a mixing machine (vertical granulator 50 L, POWREX CORPORATION), and granulated using a roller compacter (Alexanderwerk). The obtained flakes were sieved using a sieving mill (Power Mill P-3, SHOWA KAGAKU KIKAI KOUSAKUSHO CO., LTD.) to give a sized powder. To the sized powder were added crystalline cellulose (2) and magnesium stearate (2), and the mixture was mixed in a mixing machine (tumbler 60 L, SHOWA KAGAKU KIKAI KOUSAKUSHO CO., LTD.) to give granules for tableting. The granules were tableted by a rotary tableting machine (AQUARIOUS 08242L2JI, Kikusui Seisakusho Ltd.) using a 6.5 mm punch to give core tablets (weight 110 mg per tablet). On the other hand, titanium oxide, red ferric oxide, yellow ferric oxide and talc were dispersed in an aqueous hydroxypropylmethylcellulose (hypromellose) solution to prepare a film coating solution. The film coating solution was sprayed on the above-mentioned core tablets in a film coating machine (Dria Coater DRC650, POWREX CORPORATION) to give film-coated tablets containing 5 mg (94000 tablets) and 20 mg (86000 tablets) of compound (A) (free form) per tablet.

**Table 1**

| component (mg/tablet) | compound (A) free form | |
|---|---|---|
| | 5 mg | 20 mg |
| core tablet | | |
| hydrochloride of compound (A) | 5.365 | 21.46 |
| mannitol | 53.635 | 37.54 |
| crystalline cellulose (1) | 20.5 | 20.5 |
| low-substituted hydroxypropylcellulose | 9.1 | 9.1 |
| light anhydrous silicic acid | 0.35 | 0.35 |
| magnesium stearate (1) | 2.2 | 2.2 |
| crystalline cellulose (2) | 18.3 | 18.3 |
| magnesium stearate (2) | 0.55 | 0.55 |
| film coating | | |
| hypromellose | 3.3 | 3.3 |
| talc | 0.735 | 0.735 |
| titanium oxide | 0.365 | 0.365 |
| red ferric oxide | 0.025 | 0.025 |
| yellow ferric oxide | 0.05 | 0.05 |
| total | 114.475 | 114.475 |

### Example 2

According to the formulation of Table 2, hydrochloride (monohydrochloride) of compound (A), mannitol, crystalline cellulose (1), low-substituted hydroxypropylcellulose, magnesium stearate (1) and light anhydrous silicic acid were uniformly mixed in a mixing machine (vertical granulator 10 L, POWREX CORPORATION), and granulated using a roller compacter (Alexanderwerk). The obtained flakes were sieved using a sieving mill (Power Mill P-3, SHOWA KAGAKU KIKAI KOUSAKUSHO CO., LTD.) to give a sized powder. To the sized powder were added crystalline cellulose (2) and magnesium stearate (2), and the mixture was mixed in a mixing machine (tumbler 5 L, SHOWA KAGAKU KIKAI KOUSAKUSHO CO., LTD.) to give granules for tableting. The granules were tableted by a rotary tableting machine (Correct 19K, Kikusui Seisakusho Ltd.) using a 11×6 mm punch to give a core tablet (weight 220 mg). On the other hand, titanium oxide, red ferric oxide, yellow ferric oxide and talc were dispersed in an aqueous hydroxypropylmethylcellulose (hypromellose) solution to prepare a film coating solution. The film coating solution was sprayed on the above-mentioned core tablets in a film coating machine (HI-COATER LABO, Freund Corporation) to give film-coated tablets containing 40 mg of compound (A) (free form) per tablet.

**Table 2**

| component (mg/tablet) | compound (A) free form 40 mg |
|---|---|
| core tablet | |
| hydrochloride of compound (A) | 42.92 |
| mannitol | 75.58 |
| crystalline cellulose (1) | 40.3 |
| low-substituted hydroxypropylcellulose | 18.3 |
| light anhydrous silicic acid | 0.7 |
| magnesium stearate (1) | 4.4 |
| crystalline cellulose (2) | 36.7 |
| magnesium stearate (2) | 1.1 |
| film coating | |
| hypromellose | 6.6 |
| talc | 1.47 |
| titanium oxide | 0.73 |
| red ferric oxide | 0.05 |
| yellow ferric oxide | 0.1 |
| total | 228.95 |

### Example 3

According to the formulation of Table 3, hydrochloride (monohydrochloride) of compound (A), mannitol, crystalline cellulose (1), low-substituted hydroxypropylcellulose, magnesium stearate (1) and light anhydrous silicic acid were uniformly mixed in a mixing machine (vertical granulator 10 L, POWREX CORPORATION), and granulated using a roller compacter (Alexanderwerk). The obtained flakes were sieved using a sieving mill (Power Mill P-3, SHOWA KAGAKU KIKAI KOUSAKUSHO CO., LTD.) to give a sized powder. To the sized powder were added crystalline cellulose (2) and magnesium stearate (2), and the mixture was mixed in a mixing machine (tumbler 5 L, SHOWA KAGAKU KIKAI KOUSAKUSHO CO., LTD.) to give granules for tableting. The granules were tableted by a rotary tableting machine (Correct 19K, Kikusui Seisakusho Ltd.) using a 8 mm punch to give a core tablet (weight 220 mg) containing 10 mg of compound (A) (free form) per tablet.

**Table 3**

| component (mg/tablet) | compound (A) free form 10 mg |
|---|---|
| core tablet | |
| hydrochloride of compound (A) | 10.73 |
| mannitol | 107.77 |
| crystalline cellulose (1) | 40.3 |
| low-substituted hydroxypropylcellulose | 18.3 |
| light anhydrous silicic acid | 0.7 |
| magnesium stearate (1) | 4.4 |
| crystalline cellulose (2) | 36.7 |
| magnesium stearate (2) | 1.1 |
| total | 220 |

### Example 4

According to the formulation of Table 4, hydrochloride (monohydrochloride) of compound (A), mannitol, crystalline cellulose (1), low-substituted hydroxypropylcellulose, magnesium stearate (1) and light anhydrous silicic acid were uniformly mixed in a mixing machine (vertical granulator 10 L, POWREX CORPORATION), and granulated using a roller compacter (Alexanderwerk). The obtained flakes were sieved using a sieving mill (Power Mill P-3, SHOWA KAGAKU KIKAI KOUSAKUSHO CO., LTD.) to give a sized powder. To the sized powder were added crystalline cellulose (2) and magnesium stearate (2), and the mixture was mixed in a bag to give granules for tableting. The granules were tableted by a rotary tableting machine (Correct 19K, Kikusui Seisakusho Ltd.) using a 9 mm punch to give core tablets (weight 300 mg per tablet). On the other hand, titanium oxide, red ferric oxide, yellow ferric oxide and talc were dispersed in an aqueous hydroxypropylmethylcellulose (hypromellose) solution to prepare a film coating solution. The film coating solution was sprayed on the above-mentioned core tablets in a film coating machine (HI-COATER LABO, Freund Corporation) to give film-coated tablets containing 10 mg and 50 mg of compound (A) (free form) per tablet.

**[Table 4]**

| component (mg/tablet) | compound (A) 10 mg | free form 50 mg |
|---|---|---|
| core tablet | | |
| hydrochloride of compound (A) | 10.73 | 53.65 |
| mannitol | 153.77 | 110.85 |
| crystalline cellulose (1) | 55 | 55 |
| low-substituted hydroxypropylcellulose | 25 | 25 |
| light anhydrous silicic acid | 1 | 1 |
| magnesium stearate (1) | 1.5 | 1.5 |
| crystalline cellulose (2) | 50 | 50 |
| magnesium stearate (2) | 3 | 3 |
| film coating | | |
| hypromellose | 9 | 9 |
| talc | 2 | 2 |
| titanium oxide | 1 | 1 |
| red ferric oxide | 0.067 | 0.067 |
| yellow ferric oxide | 0.133 | 0.133 |
| total | 312.2 | 312.2 |

### Example 5

According to the formulation of Table 5, hydrochloride (monohydrochloride) of compound (A), mannitol, crystalline cellulose, low-substituted hydroxypropylcellulose, magnesium stearate and light anhydrous silicic acid were uniformly mixed in a mortar, and tableted by Autograph (AG-1, Shimadzu Corporation) using a 9 mm punch to give a core tablet (weight 300 mg).

**[Table 5]**

| component (mg/tablet) | compound (A) free form 50 mg |
|---|---|
| core tablet | |
| hydrochloride of compound (A) | 53.65 |
| mannitol | 100.85 |
| crystalline cellulose | 105 |
| low-substituted hydroxypropylcellulose | 25 |
| light anhydrous silicic acid | 1 |
| magnesium stearate | 4.5 |
| total | 300 |

### Experimental Example 1

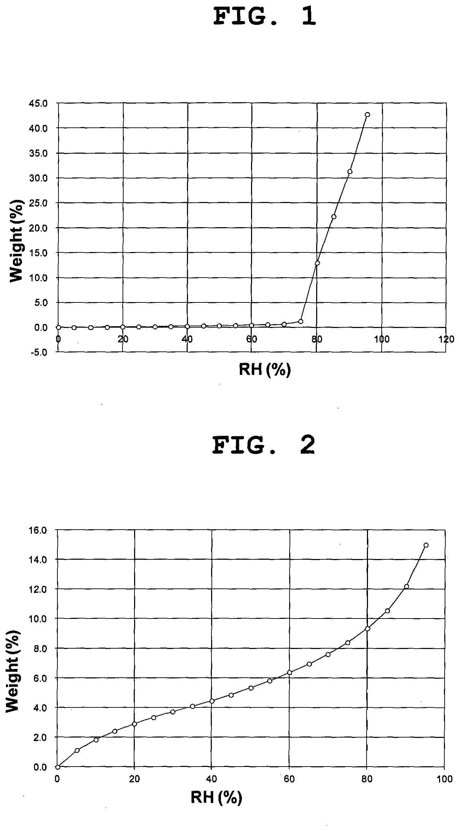

The equilibrium moisture absorption rate of hydrochloride (monohydrochloride) of compound (A) at various relative humidities at 25°C was measured by a vapor sorption analyzer (SGA-CX, VTI Corporation). The results are shown in Fig. 1.

### Experimental Example 2

The equilibrium moisture absorption rate of crystalline cellulose (CEOLUS PH-302 (Asahi Kasei Chemicals Corporation) at various relative humidities at 25°C was measured by a vapor sorption analyzer (SGA-CX, VTI Corporation). The results are shown in Fig. 2.

### Experimental Example 3

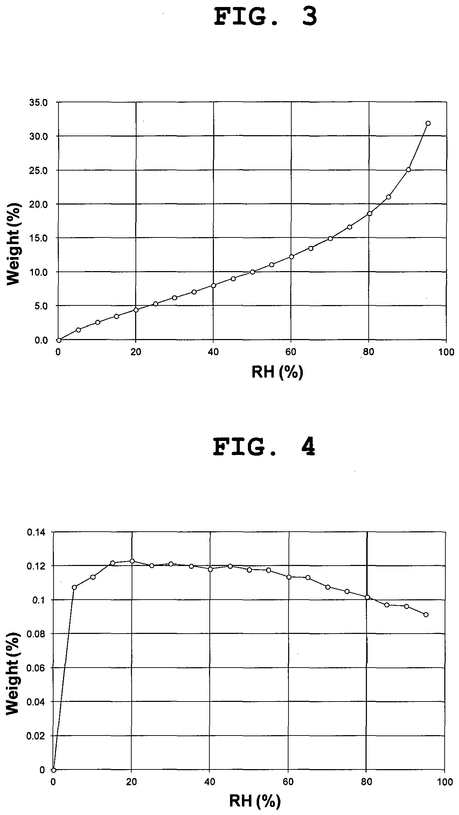

The equilibrium moisture absorption rate of low-substituted hydroxypropylcellulose (LH-21 (Shin-Etsu Chemical Co., Ltd.)) at various relative humidities at 25°C was measured by a vapor sorption analyzer (SGA-CX, VTI Corporation). The results are shown in Fig. 3.

### Example 6

According to the formulation of Table 6, hydrochloride (monohydrochloride) of compound (A), mannitol, crystalline cellulose, low-substituted hydroxypropylcellulose, magnesium stearate and light anhydrous silicic acid were uniformly mixed in a mortar, and the mixture was tableted by a desktop tableting machine (HANDTAB, ICHIHASHI-SEIKI CO., LTD.) using a 6.5 mm punch to give a core tablet (weight 110 mg).

**[Table 6]**

| component (mg/tablet) | compound (A) free form 20 mg |
|---|---|
| core tablet | |
| hydrochloride of compound (A) | 21.46 |
| mannitol | 37.54 |
| crystalline cellulose | 38.8 |
| low-substituted hydroxypropylcellulose | 9.1 |
| light anhydrous silicic acid | 0.35 |
| magnesium stearate | 2.75 |
| total | 110 |

### Comparative Example 1

According to the formulation of Table 7, hydrochloride (monohydrochloride) of compound (A), mannitol, magnesium stearate and light anhydrous silicic acid were uniformly mixed in a mortar, and the mixture was tableted by a desktop tableting machine (HANDTAB, ICHIHASHI-SEIKI CO., LTD.) using a 6.5 mm punch to give a core tablet (weight 110 mg).

**[Table 7]**

| component (mg/tablet) | compound (A) free form 20 mg |
|---|---|
| core tablet | |
| hydrochloride of compound (A) | 21.46 |
| mannitol | 85.44 |
| light anhydrous silicic acid | 0.35 |
| magnesium stearate | 2.75 |
| total | 110 |

### Experimental Example 4

The equilibrium moisture absorption rate of xylitol (reagent special grade xylitol (Wako Pure Chemical Industries, Ltd.)) at various relative humidities at 25°C was measured by a moisture adsorption and desorption measuring apparatus (SGA-CX, VTI Corporation). The results are shown in Fig. 4.

### Experimental Example 5

The tablets of Example 6 and Comparative Example 1 were preserved under the conditions of 25°C and relative humidity of 93%, and the weight loss of drying was measured under the conditions of 105°C and 3 hr. The results are shown in Table 8.

**[Table 8]**

| | Example 6 | Comparative Example 1 |
|---|---|---|
| loss on drying | 9.15% | 8.23% |

### Experimental Example 6

The tablets of Example 6 and Comparative Example 1 were preserved under the conditions of 25°C and relative humidity of 93%, and the dissolution property of compound (A) was measured according to the Japanese Pharmacopoeia Paddle Method (rotation number 50 rpm, 37°C, 0.1N hydrochloric acid test solution 900 mL, n=3). The results are shown in Table 9. The values in the Table each show an average dissolution ratio of 3 tablets.

**[Table 9]**

| | | Example 6 | | Comparative Example 1 | |
|---|---|---|---|---|---|
| | | before preservation | after preservation | before preservation | after preservation |
| dissolution ratio (%) | 0 min | 0 | 0 | 0 | 0 |
| | 5 min | 24 | 24 | 25 | 25 |
| | 10 min | 48 | 64 | 54 | 35 |
| | 20 min | 84 | 99 | 91 | 59 |
| | 30 min | 96 | 99 | 100 | 84 |
| | 45 min | 99 | 99 | 100 | 99 |
| | 60 min | 100 | 99 | 100 | 100 |

As shown in Table 9, the tablet of Comparative Example 1 showed delayed dissolution of compound (A) after preservation. In contrast, the tablet of Example 6 did not show remarkable delay in the dissolution property of compound (A) after preservation, thus confirming stability of the tablet.

### INDUSTRIAL APPLICABILITY

The present invention can provide a tablet according to the claims, which is a specific tablet containing a compound showing deliquescence at a relative humidity of not less than 80% at 25°C as a pharmaceutically active ingredient, which shows improved (reduced) hygroscopicity, can be easily produced and handled, and can be stably preserved for a long term.

This application is based on a patent application No. 2012-030987 filed in Japan.

## Claims

1. A tablet comprising the following (i)-(iii):
(i) 1-(4-methoxybutyl)-N-(2-methylpropyl)-N-[(3S,5R)-5-(morpholin-4-ylcarbonyl)piperidin-3-yl]-1H-benzimidazole-2-carboxamide or a salt thereof,
(ii) 30 to 50 wt% of crystalline cellulose in the tablet (of the weight of one tablet), and
(iii) 5 to 20 wt% of low-substituted hydroxypropylcellulose in the tablet (of the weight of one tablet).

2. The tablet according to claim 1, wherein the 1-(4-methoxybutyl)-N-(2-methylpropyl)-N-[(3S,5R)-5-(morpholin-4-ylcarbonyl)piperidin-3-yl]-lH-benzimidazole-2-carboxamide or a salt thereof is 1-(4-methoxybutyl)-N-(2-methylpropyl)-N-[(3S,5R)-5-(morpholin-4-ylcarbonyl)piperidin-3-yl]-1H-benzimidazole-2-carboxamide hydrochloride.

## Patentansprüche

1. Tablette, die folgende (i)-(iii) beinhaltet:
(i) 1-(4-Methoxybutyl)-N-(2-methylpropyl)-N-[(3S,5R)-5-(morpholin-4-ylcarbonyl) piperidin-3-yl]-1H-benzimidazol-2-carboxamid oder einem Salz davon,
(ii) 30 bis 50 Gw-% kristalliner Cellulose in der Tablette (von dem Gewicht von einer Tablette) und
(iii) 5 bis 20 Gw-% niedrig substituierter Hydroxypropylcellulose in der Tablette (von dem Gewicht von einer Tablette).

2. Tablette gemäß Anspruch 1, wobei das
1-(4-Methoxybutyl)-N-(2-methylpropyl)-N-[(3S,5R)-5-(morpholin-4-ylcarbonyl)piperidin-3 -yl]-1H-benzimidazol-2-carboxamid oder ein Salz davon
1-(4-Methoxybutyl)-N-(2-methylpropyl)-N-[(3S,5R)-5-(morpholin-4-ylcarbonyl)piperidin-3 -yl]-1H-benzimidazol-2-carboxamid-hydrochlorid ist.

## Revendications

1. Comprimé comprenant les composants (i) à (iii) suivants :
(i) du 1-(4-méthoxy-butyl)-*N*-(2-méthyl-propyl)-*N*-[(3*S*,5*R*)-5-(morpholin-4-yl-carbonyl)-pipéridin-3-yl]-1*H*-benzimidazole-2-carboxamide ou l'un de ses sels,
(ii) de la cellulose cristalline, en une proportion de 30 % à 50 % en poids dans le comprimé (pourcentage rapporté au poids d'un seul comprimé),
(iii)et de l'hydroxypropyl-cellulose à faible degré de substitution, en une proportion de 5 % à 20 % en poids dans le comprimé (pourcentage rapporté au poids d'un seul comprimé).

2. Comprimé selon la revendication 1, dans lequel le 1-(4-méthoxy-butyl)-*N*-(2-méthyl-propyl)-*N*-[(3*S*,5*R*)-5-(morpholin-4-yl-carbonyl)-pipéridin-3-yl]-1*H-*benzimidazole-2-carboxamide ou le sel de ce composé est du chlorhydrate de 1-(4-méthoxy-butyl)-*N*-(2-méthyl-propyl)-*N*-[(3*S*,5*R*)-5-(morpholin-4-yl-carbonyl)-pipéridin-3-yl]-1*H*-benzimidazole-2-carboxamide.
